(19) Europäisches Patentamt
European Patent Office
Office européen des brevets

(11) Publication number: 0 291 999
A2

(12) # EUROPEAN PATENT APPLICATION

(21) Application number: 88108126.9

(51) Int. Cl.4: C07K 7/00 , A61K 37/02

(22) Date of filing: 20.05.88

Claims for the following Contracting States: ES + GR.

(30) Priority: 21.05.87 US 53166
01.02.88 US 151060

(43) Date of publication of application:
23.11.88 Bulletin 88/47

(84) Designated Contracting States:
AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Berman, Judd M.
14 Wentworth Avenue
Cincinnati Ohio 45215(US)
Inventor: Pelton, John T.
3 rue Murner
F-67000 Strasbourg(FR)
Inventor: Hassman, Chester F., III
727 Dixmyth Avenue Nr. 309
Cincinnati Ohio 45220(US)
Inventor: Buck, Stephen H.
6818 Leeds Lane West
Cincinnati Ohio 45215(US)

(74) Representative: Macchetta, Francesco et al
Gruppo Lepetit S.p.A. Patent and Trademark Department 34, Via Roberto Lepetit
I-21040 Gerenzano (Varese)(IT)

(54) **Novel ANF derivatives.**

(57) Novel ANF derivatives, wherein one or more amino acid residues or derivatives thereof are replaced by one or more spacer groups, are described; these compounds are useful in the treatment of patients afflicted with disease states characterized by abnormalities in fluid, electrolyte, blood pressure, intraocular pressure, renin, or aldosterone homeostasis.

EP 0 291 999 A2

## NOVEL ANF DERIVATIVES

The natural regulation of extracellular fluid in animals is known to involve complex interactions of various hormonal and neurological mechanisms. Recently, a polypeptide hormone produced by the cardiac atria has been implicated as playing an important role in the homeostatic control of body sodium, water, and blood pressure. This polypeptide hormone has been termed atrial natriuretic factor (ANF) but has also been referred to as cardionatrin and atriopeptin. For the purpose of convenience this substance will be referred to herein as ANF.

A family of ANF peptides has been isolated and their amino acid sequences determined. These ANF peptides have been found to vary in length from 21 to 126 amino acids with a common structural feature being one or more disulfide-looped sequences of 17 amino acids with various amino- and carboxy- terminal sequences attached to the cystine moiety.

ANF peptides have been found to bind to specific binding sites in various tissues including kidney, adrenal, aorta, and vascular smooth muscle with affinities ranging from about 50 pico-molar (pM) to about 500 nano-molar (nM) [Needleman, Hypertension, 7, 469 (1985)]. In addition, it is believed that ANF binds to specific receptors in the brain and possibly serves as a neuromodulator as well as a conventional peripheral hormone.

The biological properties of ANF peptides involve potent diuretic / natriuretic and vasodilatory / hypotensive activity as well as an inhibitory effect on renin and aldosterone secretion [ deBold, Science 230, 767 (1985)]. It is therefore believed that ANF, and analogs thereof with ANF - like biological activity, will provide effective treatment of patients afflicted with disease states characterized by abnormalities in fluid, electrolyte, blood pressure, intraocular pressure, renin, or aldosterone homeostasis, such as, but not limited to , hypertension, renal diseases, hyperaldosteronemia, cardiac hypertrophy, glaucoma and congestive heart failure.

A biologically active ANF fragment consisting of 28 amino acids has been isolated from rat atrial tissue and has the following sequence:

(H)-$Ser_1$ - Leu - Arg - Arg - $Ser_5$ - Ser - Cys(S) - Phe - Gly- $Gly_{10}$

Ser - Gln - Ala - Gly - $Ile_{15}$ - Arg - Asp - Ile - Arg

$Gly_{20}$ - Leu - Gly - Cys(S) - Asn - $Ser_{25}$ - Phe - Arg - Tyr-(OH)

wherein the amino acid $Ser_1$ represents the amino-terminal residue, $Tyr_{28}$ represents the carboxy terminal residue, and individual amino acids are identified by their sequential numerical position as numbered from the amino terminal end to the carboxy terminal end. This peptide is referred to $rANF_{1-28}$. A corresponding peptide fragment with similar biological activity has been isolated from human atrial tissue and differs from $rANF_{1-28}$ only in that the amino acid "Ile" in position 12 is replaced by a "Met" [Needleman et al., Hypertension 7, 469 (1985)]. The corresponding human ANF peptide is referred to herein as $hANF_{1-28}$.

Applicants have now discovered certain novel compounds which effectively bind to ANF receptors and exhibit ANF-like biological effects. It is believed that these compounds are useful agents in the treatment of patients afflicted with disease states characterized by abnormalities in fluid, electrolyte, blood pressure, intraocular pressure, renin, or aldosterone homeostasis, such as, but not limited to hypertension, renal disease, hyperaldosteronemia, cardiac hypertrophy, glaucoma and congestive heart failure.

The present invention relates to novel compounds and to pharmaceutical compositions thereof. Another aspect of this invention relates to methods of use of these novel compounds in the treatment of patients

afflicted with disease states characterized by abnormalities in fluid, electrolyte, blood pressure, intraocular pressure, renin, or aldosterone homeostasis, such as, but not limited to hypertension, renal disease, hyperaldosteronemia, cardiac hypertrophy, glaucoma and congestive heart failure.

More specifically the present invention provides compounds of the formula 1

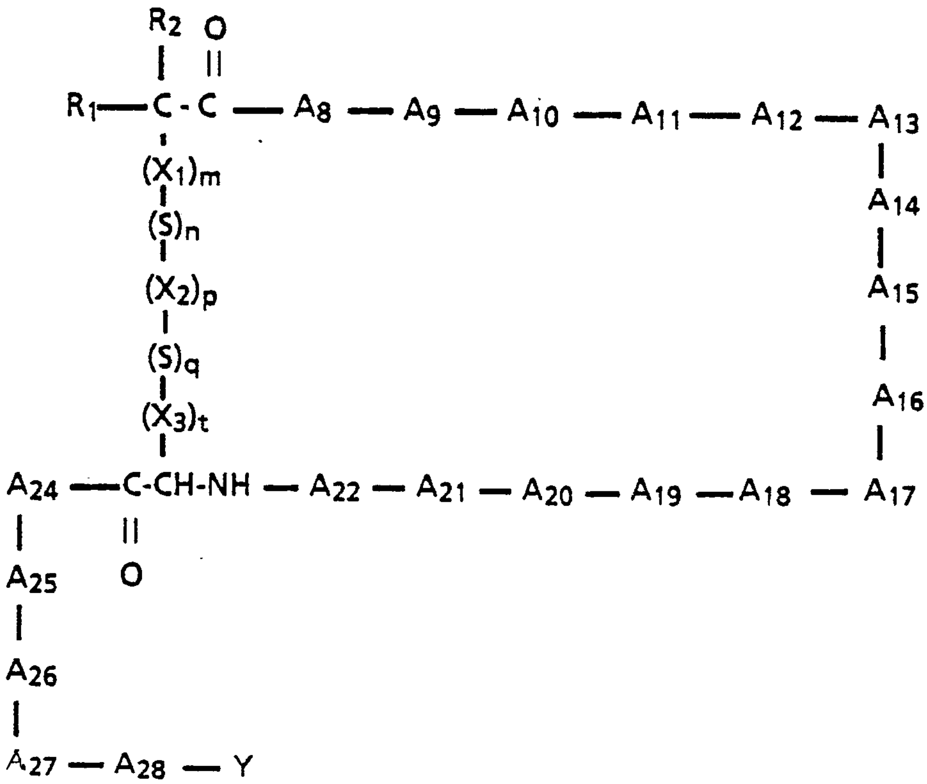

(1)

wherein,

$R_1$ is hydrogen, $C_{1-6}$ alkyl, a radical of the formula

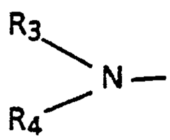

or

$R_3 — R_5 — NH —$

wherein

$R_3$ and $R_4$ are each independently hydrogen or an amino protecting group;

$R_5$ is a polypeptide of 1 to 10 amino acid residues,

$R_2$ is hydrogen or $C_{1-6}$ alkyl with the provision that when $R_1$ and $R_2$ are each alkyl they can be independent chains or they can be taken together with the carbon atom to which they are attached to form a ($C_3$-$C_{13}$) cyclic alkyl;

$X_1$, $X_2$, and $X_3$ are each independently $C_{1-8}$ alkylene radicals;

m, n, p, q, and t are each independently integers 0 or 1;

$A_8$ is a bond, Phe, N-substituted $C_{1-4}$ alkyl Phe, α-C substituted $C_{1-4}$ alkyl Phe, or D-Phe;

$A_9$ is a bond, Gly, Ala, N-substituted $C_{1-4}$ alkyl -Gly or -Ala, α-C substituted $C_{1-4}$ alkyl -Gly or -Ala, or D-Ala;

$A_{10}$ is a bond, Gly, Ala, N-substituted $C_{1-4}$ alkyl -Gly or -Ala, α-C substituted $C_{1-4}$ alkyl -Gly or -Ala, or D-Ala;

$A_{11}$ is a bond, Arg, N-substituted $C_{1-4}$ alkyl Arg, α-C-substituted $C_{1-4}$ alkyl Arg, or D-Arg;

$A_{12}$ is a bond, Ile, Nle, or Met; N-substituted $C_{1-4}$ alkyl -Ile, -Nle, or -Met; α-C-substituted $C_{1-4}$ alkyl -Ile,

-Nle, or -Met; or D-Ile, D-Nle, or D-Met;
$A_{13}$ is a bond, Asp or Glu; N-substituted $C_{1-4}$ alkyl -Asp or -Glu; α-C-substituted $C_{1-4}$ alkyl -Asp or -Glu; or D-Asp or D-Glu;
$A_{14}$ is a bond, Arg, N-substituted $C_{1-4}$ alkyl Arg, α-C-substituted $C_{1-4}$ alkyl Arg, or D-Arg;
$A_{15}$ is a bond, Ile, Nle, or Met; N-substituted $C_{1-4}$ alkyl -Ile, -Nle, or -Met; α-C-substituted $C_{1-4}$ alkyl -Ile, -Nle, or -Met; or D-Ile, D-Nle, or D-Met;
$A_{16}$ is a bond, Gly, Ala, N-substituted $C_{1-4}$ alkyl -Gly or -Ala, α-C substituted $C_{1-4}$ alkyl -Gly or -Ala, or D-Ala;
$A_{17}$ is a bond, Gly, Ala, N-substituted $C_{1-4}$ alkyl -Gly or -Ala, α-C substituted $C_{1-4}$ alkyl -Gly or -Ala, or D-Ala;
$A_{18}$ is a bond, Gln or Asn;N-substituted $C_{1-4}$ alkyl -Gln or -Asn, α-C substituted $C_{1-4}$ alkyl -Gln or -Asn, or D-Gln or D-Asn;
$A_{19}$ is a bond, Ser or Thr;N-substituted $C_{1-4}$ alkyl -Ser or -Thr; α-C substituted $C_{1-4}$ alkyl -Ser or -Thr, or D-Ser or D-Thr;
$A_{20}$ is a bond, Gly or Ala;N-substituted $C_{1-4}$ alkyl -Gly or -Ala; α-C substituted $C_{1-4}$ alkyl -Gly or -Ala; or D-Ala;
$A_{21}$ is a bond, Leu or Nle;N-substituted $C_{1-4}$ alkyl -Leu or -Nle; α-C substituted $C_{1-4}$ alkyl -Leu or -Nle; or D-Leu or D-Nle;
$A_{22}$ is a bond, Gly or Ala;N-substituted $C_{1-4}$ alkyl -Gly or -Ala; α-C substituted $C_{1-4}$ alkyl -Gly or -Ala; or D-Ala;
$A_{24}$ is a bond, Asn or Gln, N-substituted $C_{1-4}$ alkyl Asn or Gln, α-C-substituted $C_{1-4}$ alkyl Asn or Gln, or D-Asn or D-Gln;
$A_{25}$ is a bond, Ser, N-substituted $C_{1-4}$ alkyl Ser, α-C-substituted $C_{1-4}$ alkyl Ser, or D-Ser;
$A_{26}$ is a bond, Phe, N-substituted $C_{1-4}$ alkyl Phe; α-C-substituted $C_{1-4}$ alkyl Phe, or D-Phe;
$A_{27}$ is a bond, Arg, N-substituted $C_{1-4}$ alkyl Arg, α-C-substituted $C_{1-4}$ alkyl Arg, or D-Arg;
$A_{28}$ is a bond, Tyr, N-substituted $C_{1-4}$ alkyl Tyr, α-C-substituted $C_{1-4}$ alkyl Tyr, or D-Tyr;
Y is -OH, $C_{1-6}$ alkoxy, amino, mono- or di- $C_{1-4}$ alkyl substituted amino;

and wherein one or more amino acid residues, or derivatives thereof, designated as "A" above, are replaced by one or more divalent radicals of the formula

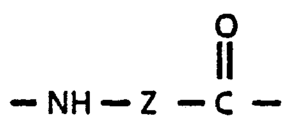

wherein Z is a $C_{2-24}$ alkylene;

with the provision that no more than 5 amino acid residues, or derivatives thereof,designated as "A" above, are bonds, and that the compounds of formula 1 are characterized by a binding constant for ANF receptors of less than about 500 nM; or pharmaceutically acceptable non-toxic salts thereof.

In another embodiment of the present invention, a method is provided for the treatment of patients afflicted with disease states characterized by abnormalities in fluid, electrolyte, blood pressure, intraocular pressure, renin, or aldosterone homeostasis, such as, but not limited to hypertension, renal disease, hyperaldosteronemia, cardiac hypertrophy, glaucoma and congestive heart failure. This method of treatment comprises administering to said patient a therapeutically effective amount of a compound of formula 1, or a pharmaceutically acceptable non-toxic salt thereof. In effecting treatment of a patient afflicted with such disease states a compound of formula 1 can be administered orally or parenterally in any manner which makes it bioavailable in effective amounts. In general, parenteral administration is preferred including for example, subcutaneous , intramuscular , intravenous , transdermal, intranasal, rectal, administration and the like.

In another embodiment of the present invention, pharmaceutical compositions are provided for administration of compounds of the formula 1. These compositions comprise a therapeutically effective amount of one or more compounds of the formula 1 in with one or more pharmaceutically acceptable carriers.

The following common abbreviations of amino acids are used throughout this specification:
Ala - alanine
Asn - asparagine

Asp - aspartic acid
Arg - arginine
Cys - cysteine
Gln - glutamine
Glu - glutamic acid
Gly - glycine
Ile - isoleucine
Leu - leucine
Met - methionine
Nle - norleucine
Phe - phenylalanine
Sar- sarcosine (N-methyl glycine)
Ser - serine
Thr - threonine
Tyr - tyrosine

It is understood that in referring to any particular amino acid listed above as part of a peptide molecule herein the divalent radical of that amino acid is meant. For example, "Gly" refers to the divalent radical of the formula

$$-NH-CH_2-\overset{\displaystyle O}{\overset{\|}{C}}-$$

The amino acids listed above, with the exception of glycine and sarcosine, are optically active because of the chiral nature of their α-carbon atom. Naturally-occurring amino acids are of the L-configuration. It is understood that reference herein to a particular amino acid refers to the L-configuration of that amino acid unless the D-configuration is specifically designated. For example, as used herein, Ser refers to the L-configuration of serine, whereas D-Ser is used to refer to the corresponding D-configuration.

As is customary in the art, the structures of peptides herein are generally presented such that the amino terminal end is to the viewer's left and the carboxy terminal end is to the viewer's right. Unless otherwise indicated, it is understood that peptides referred to herein are made up of the designated amino acid radicals linked through conventional peptide bonds as is well known and appreciated in the art.

As used herein, the terms "alkyl" and "alkylene" refer to hydrocarbyl and hydrocarbylene radicals of single or double valency, respectively. For example, $CH_3CH_2$- is an alkyl radical and $-CH_2CH_2-$ is the corresponding alkylene radical. Alkyl and alkylene radicals, and the alkyl/alkylene portion of an alkoxy or acyl group, are understood to include within their scope saturated and unsaturated alkyl/alkylene groups of straight, branched, or cyclic configurations including, but not limited to, methyl, ethyl, ethenyl, propyl, isopropyl, butyl, butenyl, isobutyl, tert-butyl, pentyl, isopentyl, sec-pentyl, cyclopentyl, hexyl, isohexyl, cyclohexyl, cyclohexylmethyl, heptyl, octyl, nonyl, decyl, undecyl, $CH_3-(CH_2)_{12}-CH_2-$, $CH_3-(CH_2)_{15}-CH_2-$, $CH_3-(CH_2)_{18}-CH_2-$, $CH_3-(CH_2)_{21}-CH_2-$and the like, and the corresponding alkylene radicals. ($C_3$-$C_{13}$)cyclic alkyl includes straight or branched cycloalkyl groups of from 3 to 13 carbon atoms. A preferred group of these cycloalkyl substituents is represented by ($C_5$-$C_7$)cycloalkyl groups. An alkoxy radical is understood to include within its scope hydroxy-substituted alkyl/alkylene groups incorporated into the compounds of formula 1 as carboxylic acid esters including, but not limited to, methoxy, ethoxy, propyloxy, isopropyloxy, butyloxy, pentyloxy, hexyloxy and the like, and the corresponding alkylene radicals. An acyl group is understood to include within its scope groups having one or two carbonyl moieties per group along with the specified alkyl/alkylene radical including, but not limited to, acetyl, benzoyl, and succinyl, and the like. As used herein, the designation "S" refers to a sulfur atom.

In compounds of the formula 1, one or more amino acids designated as "A" are replaced with one or more divalent radicals of the general formula

$$-NH-Z-\overset{\displaystyle O}{\overset{\|}{C}}-$$

wherein Z is a $C_{2-24}$ alkylene.

Generally, in compounds of the formula 1, amino acid residues are replaced individually or groups of contiguous amino acid residues are replaced as a unit. The above identified divalent radicals, which are referred to herein as "spacer" groups, are amino-substituted carboxylic acids and are incorporated into the compounds of the formula 1 through conventional peptide bonds. The moiety Z is a saturated or unsaturated alkylene moiety of straight, branched, or cyclic configuration,which is made up of from 2 to 24 carbon atoms. It is generally preferred to replace an amino acid or a group of contiguous amino acids with a spacer moiety of approximately the same N- to carboxy-terminal length as that from the α-N terminus to the α- carboxy terminus of the replaced amino acid or group of contiguous amino acids so as to retain the same approximate spacial relationships among the amino acid residues which are not so replaced. For example, it is preferred to replace a group of 2 contiguous amino acids with the divalent radical of 4-aminobutyric acid or 5-aminopentanoic acid; to replace a group of 3 contiguous amino acids with the divalent radical of 7-aminoheptanoic acid or 8-aminooctanoic acid; to replace 4 contiguous amino acids with the divalent radical of 10-aminodecanoic acid or 11-amino undecanoic acid ; and so on. Although compounds so structured are generally preferred, it is understood that the present invention is not limited thereto, and that compounds in which the spacial relationship among unreplaced amino acids are not maintained, are also within the scope of the present invention.

When it is desired to replace two or more contiguous amino acids with a spacer group it is preferred to replace the group with a single spacer group. However it is contemplated by the present invention that a group of contiguous amino acids can alternatively be replaced by two or more spacer groups which are themselves linked together by peptide bonds. For example, although it is preferred to replace a group of three contiguous amino acids with an 8-aminooctanoic moiety, these residues can also be replaced by two contiguous 3-aminopropanoic acid moieties, or one 2-aminoacetic acid moiety and one 4-aminobutyric acid moiety. Of course, compounds of the formula 1 in which more than one spacer group have been incorporated are within the scope of the present invention. Compounds of formula 1 wherein one to four spacer groups have been incorporated are generally preferred.

The present invention provides for compounds of formula 1 wherein any of the amino acids designated as "A" can be absent, i.e., can be a bond. However this aspect of the present invention is limited by the provision that no more than 5 such amino acids can be absent in any compound claimed.

In its broadest generic scope, the present invention provides compounds wherein any of the amino acids designated as "A" can be replaced by spacer groups. However, it is believed that in certain sections of the compounds the amino acid residues, or derivatives thereof, should be present to facilitate effective binding to ANF receptors. It is believed that the sections of the compounds of the present invention which facilitate binding to ANF receptors comprise the section from about $A_{11}$ to about $A_{15}$ and the section at $A_{27}$. Therefore compounds of formula 1 wherein the peptide moiety identified as $A_{11}$ to $A_{15}$ and the amino acid moiety identified as $A_{27}$ are not bonds and are not replaced by spacer groups, are generally preferred embodiments of the present invention.

As with any generic group of chemical compounds, certain groups are preferred at the various positions in the molecule. Applicants prefer the compounds of formula 1 wherein $R_1$ is hydrogen or amino. Also preferred are those compounds of formula 1 wherein $R_1$ is

H-Ser-NH-;
H-Ser-Ser-NH-;
H-Arg-Ser-Ser-NH-;
H-Arg-Arg-Ser-Ser-NH-;
H-Leu-Arg-Arg-Ser-Ser-NH-;
H-Ser-Leu-Arg-Arg-Ser-Ser-NH-.

These are compounds for which $R_3$ is hydrogen and $R_5$ is the amino acid or peptide named above.

In addition, $R_3$ and $R_4$ can each be a conventional amino protecting group as is customary and well known in the art. These amino protecting groups are those which are known to be useful in the art of chemistry for protecting a primary amino group during chemical synthetic steps and which are readily

removable under conditions which will not cause degradation of the compound. Examples of suitable protecting groups for $R_3$ and $R_4$ are formyl, acetyl, trifluoroacetyl, phthalyl, tosyl, benzenesulfonyl, benzyloxycarbonyl, substituted-benzyloxycarbonyl (e.g., p-chloro, p-bromo, p-nitro, p-methoxy, o-chloro, 2,4-dichloro, and 2,6-dichloro derivatives), t-butyloxycarbonyl (Boc), t-amyloxycarbonyl, isopropyloxycarbonyl, 2-(p-biphenyl)-isopropyloxycarbonyl, allyloxycarbonyl, cyclopentyloxycarbonyl, cyclohexyloxycarbonyl, adamentyloxycarbonyl, phenylthiocarbonyl, and triphenylmethyl. The preferred amino protecting group is t-butyloxycarbonyl (Boc).The selection, utilization, and synthesis involved for particular protecting groups represented by $R_3$ and $R_4$ are well known in the art of chemistry.

Applicants also prefer those compounds of formula 1 wherein the divalent radical linkage identified as --$(X_1)_m$-$(S)_n$-$(X_2)_p$-$(S)_q$-$(X_3)_t$- is

a. -$CH_2$-S-S-$CH_2$- (i.e., wherein $X_1$ and $X_3$ are methylene; p is zero; and m,n,q,t are each one);

b. -$CH_2$-$(CH_2)_2$-$CH_2$- (i.e., wherein $X_1$ and $X_3$ are methylene; $X_2$ is ethylene; n and q are zero; and m,p,t are one);

c. 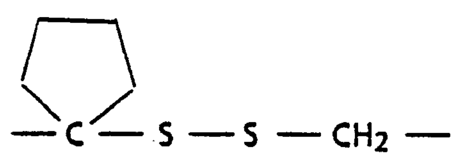

(i.e., wherein $X_1$ is 1,1-cyclopentylene and $X_3$ is methylene ;p is zero; and m,n,q,t are one);

d.-$CH_2$-S-$CH_2$-$CH_2$-S-$CH_2$- (i.e., wherein $X_1$ and $X_3$ are methylene; $X_2$ is ethylene; m,n,p,q,t are one);

e.-$C(CH_3)_2$-S-S-$C(CH_3)_2$- (i.e., wherein $X_1$ and $X_3$ are isopropylene; p is zero; m,n,q,t are one);

f.-$CH_2$-$CH_2$-S-S-$CH_2$-$CH_2$- (i.e., wherein $X_1$ and $X_3$ are ethylene; p is zero; m,n,q,t are one);

Also generally preferred are those compounds of formula 1 wherein the following amino acid residues, when not replaced by a spacer group, are present at the indicated positions:

$A_8$ is Phe, N-substituted $C_{1-4}$ alkyl Phe, or D-Phe;
$A_9$ is Gly or Ala, N-substituted $C_{1-4}$ alkyl -Gly or -Ala, or D-Ala;
$A_{10}$ is Gly;
$A_{11}$ is Arg;
$A_{12}$ is Ile, Nle, or Met; N-substituted $C_{1-4}$ alkyl -Ile, -Nle, -Met; D-Ile, D-Nle, D-Met;
$A_{13}$ is Asp, or Glu;
$A_{14}$ is Arg;
$A_{15}$ is Ile, Nle, or Met;N-substituted $C_{1-4}$ alkyl -Ile, -Nle, -Met; D-Ile, D-Nle, D-Met;
$A_{16}$ is Gly or Ala, N-substituted $C_{1-4}$ alkyl -Gly or -Ala, or D-Ala
$A_{17}$ is Ala, N-substituted $C_{1-4}$ alkyl Ala, or D-Ala.;
$A_{18}$ is Gln or Asn;
$A_{19}$ is Ser or Thr;
$A_{20}$ is Gly or Ala;
$A_{21}$ is Leu, N-substituted $C_{1-4}$ alkyl Leu, or D-Leu;
$A_{22}$ is Gly or Ala;
$A_{24}$ is Asn or Gln;
$A_{25}$ is Ser;
$A_{26}$ is Phe, N-substituted $C_{1-4}$ alkyl Phe, or D-Phe;
$A_{27}$ is Arg, N-substituted $C_{1-4}$ alkyl Arg, D-Arg;
$A_{28}$ is Tyr, N-substituted $C_{1-4}$ alkyl Tyr, D-Tyr;

The compounds of formula 1 specifically identified in Table 1 are particularly preferred embodiments of the present invention. Table 1 however does not provide an exclusive list of those compounds which are within the scope of the present invention but merely provides a representative list of particularly preferred compounds.

TABLE 1

Apa refers to 5-aminopentanoic acid
Aoa refers to 8-aminooctanoic acid
Aua refers to 11-aminoundecanoic acid
N-Me refers to N-methyl (For example, N-Me-$Ile_{15}$ refers to N-methyl isoleucine at position 15).
ANF refers to rANF or hANF.
For each compound named, Y is amino, hydroxy, methoxy or ethoxy.

The "Basic Structure" indicates that these compounds of Formula 1 are made up of amino acids identical to rANF or hANF at the positions indicated by the subscript. For example, "$ANF_{8,10-28}$" indicates that the compound is made up of amino acids identical to those known for rANF or hANF at positions $A_8$ and $A_{10}$ through $A_{28}$.$R_1$, $R_2$, and $(X_1)_m-(S)_n-(X_2)_p-(S)_q-(X_3)_t$ are specifically named in the Table. The identity of the spacer groups is indicated and their positions are identified by superscript. For example, "$Aoa^{17-19,20-22}$" indicates that the compound contains two 8-aminooctanoic acid spacer groups, one at positions $A_{17}$ through $A_{19}$ and one at positions $A_{20}$ through $A_{22}$.

TABLE 1

| Cmpd. No. | Basic Structure | $R_1$ | $R_2$ | $(X_1)_m$-$(S)_n$-$(X_2)_p$-$(S)_q$-$(X_3)_t$- | spacer groups |
|---|---|---|---|---|---|
| 1 | $ANF_{11-28}$ | H-Ser-Ser-NH- | H | $-CH_2-S-S-CH_2-$ | $Aoa^{8-10}$ |
| 2 | $ANF_{8,10-28}$ | H-Ser-Ser-NH- | H | $-CH_2-S-S-CH_2-$ | $Aoa^{9-11}$ |
| 3 | $ANF_{8-14,18-28}$ | H-Ser-Ser-NH- | H | $-CH_2-S-S-CH_2-$ | $Aoa^{15-17}$ |
| 4 | $ANF_{8-15,19-28}$ | H-Ser-Ser-NH- | H | $-CH_2-S-S-CH_2-$ | $Aoa^{16-18}$ |
| 5 | $ANF_{8-16,20-28}$ | H-Ser-Ser-NH- | H | $-CH_2-S-S-CH_2-$ | $Aoa^{17-19}$ |
| 6 | $ANF_{8-19,23-28}$ | H-Ser-Ser-NH- | H | $-CH_2-S-S-CH_2-$ | $Aoa^{20-22}$ |
| 7 | $ANF_{8-23,27-28}$ | H-Ser-Ser-NH- | H | $-CH_2-S-S-CH_2-$ | $Aoa^{24-26}$ |
| 8 | $ANF^{8-16,23-28}$ | H-Ser-Ser-NH- | H | $-CH_2-S-S-CH_2-$ | $Aoa^{17-19,20-22}$ |
| 9 | $ANF_{11-16,23,27-28}$ | H-Ser-Ser-NH- | H | $-CH_2-S-S-CH_2-$ | $Aoa^{8-10,17-19,20-22,24-26}$ |
| 10 | $ANF_{11-16,23,27-28}$ | H | H | $-CH_2-S-S-CH_2-$ | $Aoa^{8-10,17-19,20-22,24-26}$ |
| 11 | $ANF_{8,11-15,23,27-28}$ | H-Ser-Ser-NH- | H | $-CH_2-S-S-CH_2-$ | $Apa^{9-10}$,$Aoa^{16-18,24-26}$,$Aua^{19-22}$ |
| 12 | $ANF_{8-23,27-28}$ | H-Ser-Leu-Arg-Arg-Ser-Ser-NH- | H | $-CH_2-S-S-CH_2-$ | $Aoa^{24-26}$ |
| 13 | $ANF_{8-23,27-28}$ | H-Leu-Arg-Arg-Ser-Ser-NH- | H | $-CH_2-S-S-CH_2-$ | $Aoa^{24-26}$ |
| 15 14 | $ANF_{8-23,27-28}$ | H-Arg-Arg-Ser-Ser-NH- | H | $-CH_2-S-S-CH_2-$ | $Aoa^{24-26}$ |
| 15 | $ANF_{8-23,27-28}$ | H-Arg-Ser-Ser-NH- | H | $-CH_2-S-S-CH_2-$ | $Aoa^{24-26}$ |
| 16 | $ANF_{8-23,27-28}$ | H-Ser-NH- | H | $-CH_2-S-S-CH_2-$ | $Aoa^{24-26}$ |

TABLE 1

| Cmpd. No. | Basic Structure | $R_1$ | $R_2$ | $(X_1)_m$-$(S)_n$-$(X_2)_p$-$(S)_q$-$(X_3)_t$- | spacer groups |
|---|---|---|---|---|---|
| 17 | $ANF_{8-23,27-28}$ | $NH_2$- | H | -$CH_2$-S-S-$CH_2$- | $Aoa^{24-26}$ |
| 18 | $ANF_{8-23,27}$ | H-Ser-Ser-NH- | H | -$CH_2$-S-S-$CH_2$- | $Aoa^{24-26}$ |
| 19 | $ANF_{8-23}$;D-$Arg_{27}$ | H-Ser-Ser-NH- | H | -$CH_2$-S-S-$CH_2$- | $Aoa^{24-26}$ |
| 20 | $ANF_{8-23,28}$;D-$Arg_{27}$ | H-Ser-Ser-NH- | H | -$CH_2$-S-S-$CH_2$- | $Aoa^{24-26}$ |
| 21 | $ANF_{8-23,27-28}$ | H-Ser-Ser-NH- | H | -$CH_2$-$CH_2$-S-S-$CH_2$-$CH_2$- | $Aoa^{24-26}$ |
| 22 | $ANF_{8-23,27-28}$ | H-Ser-Ser-NH- | H | -$CH_2$-$CH_2$-$CH_2$-$CH_2$- | $Aoa^{24-26}$ |
| 23 | $ANF_{8-23,27-28}$ | H-Ser-Ser-NH- | H | -$C(CH_3)_2$-S-S-$C(CH_3)_2$- | $Aoa^{24-26}$ |
| 24 | N-Me-$Phe_8$;$ANF_{9-16,23,27-28}$ | H-Ser-Ser-NH- | H | -$CH_2$-S-S-$CH_2$- | $Aoa^{17-19,20-22,24-26}$ |
| 25 | $ANF_{11-14,16,18-19,23-25,27-28}$; N-Me-$Ile_{15}$;N-Me-$Ala_{17}$;N-Me-$Phe_{26}$ | H-Ser-Ser-NH- | H | -$CH_2$-S-S-$CH_2$- | $Aoa^{8-10,20-22}$ |
| 26 | $ANF_{11-14,23}$;N-Me-$Ile_{15}$;D-$Arg_{27}$ | H | H | -$CH_2$-S-S-$CH_2$- | $Aoa^{8-10,16-18,24-26}$;$Aua^{19-22}$ |
| 27 | $ANF_{11-14,23-25}$;N-Me-$Phe_{8,26}$;N-Me-$Ile_{15}$;D-$Arg_{27}$ | H | H | -$CH_2$-S-S-$CH_2$- | $Apa^{9-10}$;$Aoa^{16-18}$;$Aua^{19-22}$ |
| 28 | $ANF_{11-14,16,23-26}$;N-Me-$Phe_{8,26}$;N-Me-$Ile_{15}$;N-Me-$Ala_{17}$;D-$Arg_{27}$ | H-Ser-Ser-NH- | H | -$CH_2$-S-S-$CH_2$- | $Apa^{9-10,18-19}$;$Aoa^{20-22}$ |

TABLE 1

| Cmpd. No. | Basic Structure | $R_1$ | $R_2$ | $(X_1)_m-(S)_n-(X_2)_p-(S)_q-(X_3)_t-$ | spacer groups |
|---|---|---|---|---|---|
| 29 | $ANF_{9-16,23,27-28}$;D-$Phe_8$ | H-Ser-Ser-NH- | H | $-CH_2-S-S-CH_2-$ | $Aoa^{17-19,20-22,24-26}$ |
| 30 | $ANF_{11-14,16,18-19,23-25,27-28}$;D-$Ile_{15}$;D-$Ala_{17}$;D-$Phe_{26}$ | H-Ser-Ser-NH- | H | $-CH_2-S-S-CH_2-$ | $Aoa^{8-10,20-22}$ |
| 31 | $ANF_{11-14,23}$;D-$Ile_{15}$;D-$Arg_{27}$ | H | H | $-CH_2-S-S-CH_2-$ | $Aoa^{8-10,16-18,24-26}$;$Aua^{19-22}$ |
| 32 | $ANF_{11-14,23-25}$;D-$Phe_{8,26}$;D-$Ile_{15}$;D-$Arg_{27}$ | H | H | $-CH_2-S-S-CH_2-$ | $Apa^{9-10}$;$Aoa^{16-18}$;$Aua^{19-22}$ |
| 33 | $ANF_{11-14,16,23-25}$;D-$Phe_{8,26}$;D-$Ile_{15}$;D-$Ala_{17}$;D-$Arg_{27}$ | H-Ser-Ser-NH- | H | $-CH_2-S-S-CH_2-$ | $Apa^{9-10,18-19}$;$Aoa^{20-22}$ |
| 34 | $ANF_{8-16,23-28}$ | H-Ser-Ser-NH- | H | $-CH_2-S-S-CH_2-$ | $Aoa^{17-19,20-22}$ |
| 35 | $ANF_{8-24,28}$ | H-Ser-Ser-NH- | H | $-CH_2-S-S-CH_2-$ | $Aoa^{25-27}$ |
| 36 | $ANF_{8,11,13-14,23}$;N-Me-$Ile_{12,15}$;D-$Arg_{27}$ | H | H | $-CH_2-S-S-CH_2-$ | $Apa^{9-10}$;$Aoa^{20-22,24-26}$;$Aua^{16-19}$ |
| 37 | $ANF_{8-11,13-14,23,28}$;N-Me-$Ile_{12,15}$;D-$Arg_{27}$ | H | H | $-CH_2-S-S-CH_2-$ | $Aoa^{20-22,24-26}$;$Aua^{16-19}$ |
| 38 | $ANF_{9-11,13-14,23,28,}$;N-Me-$Phe_8$;N-Me-$Ile_{12,15}$;D-$Arg_{27}$ | H-Ser-Ser-NH- | H | $-CH_2-S-S-CH_2-$ | $Aoa^{20-22,24-26}$;$Aua^{16-19}$ |

The compounds of this invention can be prepared by a variety of procedures readily known to those skilled in the art. Such procedures include solid phase sequential and block synthesis, solution phase sequential and block synthesis, gene cloning and combinations of these techniques. The solid phase sequential procedure can be performed using established automated methods such as by use of an automated peptide synthesizer. In this procedure an amino protected amino acid is bound to a resin support at the carboxy terminal end, the amino acid is deprotected at the amino position at which a peptide linkage

is desired, and the next amino protected amino acid in the desired sequence is coupled in a peptide linkage. The deprotection and coupling steps are repeated until the desired polypeptide is synthesized. The compounds of the present invention are thus synthesized from their carboxy terminal end to their amino terminal end. The amino protected amino acid can be a conventional amino acid, a derivative or isomer thereof, or a spacer group. The resin support employed can be any suitable resin conventionally employed in the art for the solid phase preparation of polypeptides. The preferred resin is polystyrene which has been cross-linked with from about 0.5 to about 3 percent divinyl benzene, which has been either benzhydrylamidated, chloromethylated or hydroxymethylated to provide sites for amide or ester formation with the initially introduced amino protected amino acid.

An example of a hydroxymethyl resin is described by Bodansky et al. [Chem. Ind. (London) 38, 1597-98 (1966)]. The preparation of chloromethyl and benzhydrylamine resins are described by Stewart et al. ["Solid Phase Peptide Synthesis", 2nd Edition, Pierce Chemical Co., Rockford, Illinois (1984), Chapter 2, pp. 54-55]. Many of these resins are available commercially. In general, the amino protected amino acid which is desired on the carboxy-terminal end of the peptide is bound to the resin using standard procedures and practices as are well known and appreciated in the art. For example, the amino protected amino acid can be bound to the resin by the procedure of Gisin [ Helv. Chem. Acta, 56, 1476 (1973)]. When it is desired to use a resin containing a benzhydrylamine moiety as the resin binding site an amino protected amino acid is coupled to the resin through an amide linkage between its α-carboxylic acid and the amino moiety of the resin. This coupling is effected using standard coupling procedures as described below. Many resin-bound amino acids are available commercially.

The α-amino protecting group employed with each amino acid introduced into the polypeptide sequence may be any such protecting group known in the art. Among the classes of amino protecting groups contemplated are: (1) acyl type protecting groups such as formyl, trifluoroacetyl, phthalyl, p-toluenesulfonyl (tosyl), benzenesulfonyl, nitrophenylsulfenyl, tritylsulfenyl, o-nitrophenoxyacetyl, and α-chlorobutyryl; (2) aromatic urethane type protecting groups such as benzyloxycarbonyl and substituted benzyloxycarbonyls such as p-chlorobenzyloxycarbonyl, p-methoxybenzyloxycarbonyl, p-nitrobenzyloxycarbonyl, p-bromobenzyloxycarbonyl, 1-(p-biphenylyl)-1-methylethoxycarbonyl, α-,α-dimethyl-3,5-dimethoxybenzyloxycarbonyl, and benzhydryloxycarbonyl; (3) aliphatic urethane protecting groups such as tert-butyloxycarbonyl (Boc), diisopropylmethoxycarbonyl, isopropyloxycarbonyl, ethoxycarbonyl, and allyloxycarbonyl; (4) cycloalkyl urethane type protecting groups such as cyclopentyloxycarbonyl, adamantyloxycarbonyl, and cyclohexyloxycarbonyl; (5) thio urethane type protecting groups such as phenylthiocarbonyl; (6) alkyl type protecting groups such as triphenylmethyl (trityl) and benzyl (Bzl); (7) trialkylsilane protecting groups such as trimethylsilane. The preferred α-amino protecting group is tert-butyloxycarbonyl (Boc). The use of Boc as an α-amino protecting group for amino acids is described by Bodansky et al. in "The Practice of Peptide Synthesis", Springer-Verlag, Berlin (1984), p. 20.

Following the coupling of the amino protected amino acid to the resin support, the α-amino protecting group is removed using any suitable procedure such as by using trifluoroacetic acid, trifluoroacetic acid in dichloromethane, or HCl in dioxane. The deprotection is carried out at a temperature of between 0 °C and room temperature. Other standard cleaving reagents may be used for removal of specific amino protecting groups under conditions well known and appreciated in the art.

After removal of the α-amino protecting group the next desired amino protected amino acid is coupled through a peptide linkage. This deprotection and coupling procedure is repeated until a polypeptide of the desired sequence is obtained. Alternatively, multiple amino acid groups may be coupled by the solution method prior to coupling with the resin supported amino acid sequence.

The selection and use of an appropriate coupling reagent is within the skill of the ordinary practitioner in the art. Particularly suitable coupling reagents where the amino acid to be added is Gln, Asn, or Arg are N,N-dicyclohexylcarbodiimide and 1-hydroxybenzotriazole. The use of these reagents prevents nitrile and lactam formation. Other coupling agents are (1) carbodiimides (e.g., N,N′-dicyclohexylcarbodiimide and N-ethyl-N′-(γ-dimethylaminopropylcarbodiimide); (2) cyanamides (e.g., N,N-dibenzylcyanamide); (3) ketenimines; (4) isoxazolium salts (e.g., N-ethyl-5-phenylisoxazolium-3-sulfonate); (5) monocyclic nitrogen containing heterocyclic amides of aromatic character containing one through four nitrogens in the ring such as imidazolides, pyrazolides, and 1,2,4-triazolides (specific heterocyclic amides that are useful include N,N′-carbonyldiimidazole and N,N′-carbonyl-di-1,2,4-triazole); (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-o-Ala-Boc); (8) nitrogen containing heterocyclic compounds having a hydroxy group on one ring nitrogen (e.g., N-hydroxyphthalimide, N-hydroxysuccinimide, and 1-hydroxybenzotriazole). Other activating reagents and their use in peptide coupling are described by Kapoor [J. Pharm. Sci., 59, 1-27 (1970)]. The

generally preferred coupling method for the amino acids used in the present invention is the use of the symmetrical anhydride as the coupling agent.

The preferred coupling method for Gln, Asn, and Arg is to react the protected amino acid, or derivatives or isomers thereof, with N,N-dicyclohexylcarbodiimide and 1-hydroxybenzotriazole (1:1) in N,N-dimethylformamide (DMF) in the presence of the resin or resin-bound amino acid or peptide. The preferred coupling method for other amino acids or spacer groups involves reacting the protected amino acid, or derivative or isomer thereof, with N,N-dicyclohexylcarbodiimide in dichloromethane to form the symmetrical anhydride. The symmetrical anhydride is then introduced into the solid phase reactor containing the resin or resin-bound amino acid or peptide, and the coupling is carried out in a medium of (DMF), or dichloromethane, or DMF : dichloromethane (1:1).A medium of DMF is preferred. The success of the coupling reaction at each stage of the synthesis is monitored by a ninhydrin test as described by Kaiser et al. [ Analyt. Biochem. 34, 595 (1970)]. In cases where incomplete coupling occurs, the coupling procedure is repeated. If the coupling is still incomplete, the deprotected amine is capped with a suitable capping reagent to prevent its continued synthesis. Suitable capping reagents and the use thereof are well known and appreciated in the art. Examples of suitable capping reagents are acetic anhydride and acetylimidazole as described by Stewart et al. ["Solid Phase Peptide Synthesis", 2nd Ed., Pierce Chemical Co., Rockford, Ill. (1984), Chapter 2, p. 73].

After the desired amino acid sequence has been obtained, the peptide is cleaved from the resin. This can be effected by procedures which are well known and appreciated in the art, such as by hydrolysis of the ester or amide linkage to the resin. It is preferred to cleave the peptide from the benzhydrylamine resin with a solution of dimethyl sulfide, p-cresol, thiocresol, or anisole in anhydrous hydrogen fluoride. The cleavage reaction is preferrably carried out at temperatures between about zero degrees Celsius (0 °C) and about room temperature, and is allowed to continue preferrably from between about 5 minutes to about 5 hours.

As is known in the art of solid phase peptide synthesis, many of the amino acids bear side chain functionalities requiring protection during the preparation of the peptide. The selection and use of an appropriate protecting group for these side chain functionalities is within the ability of those skilled in the art and will depend upon the amino acid to be protected and the presence of other protected amino acid residues in the peptide. The selection of such a side chain protecting group is critical in that it must not be removed during the deprotection and coupling steps of the synthesis. For example, when Boc is used as the α-amino protecting group, the following side chain protecting groups are suitable: p-toluenesulfonyl (tosyl) moieties can be used to protect the amino side chains of amino acids such as Lys and Arg; p-methylbenzyl, acetamidomethyl, benzyl (Bzl), or t-butylsulfonyl moieties can be used to protect the sulfide containing side chains of amino acids such as cysteine, homocysteine, penicillamine and the like or derivatives thereof ;benzyl (Bzl) or cyclohexyl ester moieties can be used to protect carboxylic acid side chains of amino acids such as Asp, Glu; a benzyl (Bzl) ether can be used to protect the hydroxy containing side chains of amino acids such as Ser and Thr; and a 2-bromocarbobenzoxy (2Br-Z) moiety can be used to protect the hydroxy containing side chains of amino acids such as Tyr. These side chain protecting groups are added and removed according to standard practices and procedures well known in the art. It is preferred to deprotect these side chain protecting groups with a solution of anisole in anhydrous hydrogen fluoride (1:10). Typically, deprotection of side chain protecting groups is performed after the peptide chain synthesis is complete but these groups can alternatively be removed at any other appropriate time. It is preferred to deprotect these side chains at the same time as the peptide is cleaved from the resin.

After cleaving the peptide from the resin, the bridge identified herein as $-(X_1)_m-(S)_n-(X_2)_p-(S)_q-(X_3)_r-$ is formed by covalently linking the side chains of the appropriate moieties according to standard procedures and practices as are well known in the art. For example, disulfide linkages such as $-CH_2-S-S-CH_2-$, $-CH_2-CH_2-S-S-CH_2-CH_2-$, and $-C(CH_3)_2-S-S-C(CH_3)_2-$,are effected by oxidation of the appropriate sulfides as described by Stewart et al. ["Solid Phase Peptide Synthesis", 2nd Edition, Pierce Chemical Co., Rockford, Ill., (1984), p.95]. Alkyl linkages such as $-CH_2-(CH_2)_2-CH_2-$ are effected as described in U.S. Patent No. 4,161,521. Linkages wherein p is one , such as $-CH_2-S-CH_2-CH_2-S-CH_2-$, are effected as described by Mosberg et al. [JACS 107, 2986-87 (1985)]. The descriptions of the above procedures are incorporated herein by reference.

The desired amino acids, derivatives and isomers thereof, and spacer groups can be obtained commercially or can be synthesized according to standard practices and procedures well known in the art.

In order to more fully illustrate the preparation of compounds of this invention, the following examples are provided. The examples are illustrative only and are not intended to limit the scope of the invention in any way.

## EXAMPLE 1

PREPARATION OF $[Aoa^{24-26}]rANF_{5-23,27-28}-NH_2$

$[Aoa^{24-26}]rANF_{5-28}-NH_2$ is represented structurally as follows:

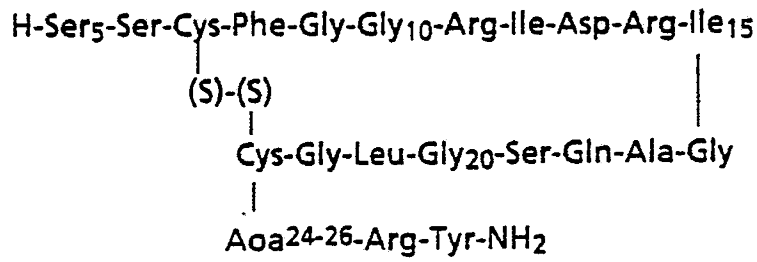

i.e., the basic structure is $rANF_{5-23,27-28}$; $R_1$ is H-Ser-Ser-NH-; $R_2$ is H; $(X_1)_m-(S)_n-(X_2)_p-(S)_q-(X_3)_t-$ is $-CH_2-S-S-CH_2-$; the spacer group is $Aoa^{24-26}$; and Y is $-NH_2$.

The synthesis of this peptide is based on standard solid-phase peptide synthesis methodology using an automated peptide synthesizer supplied by Applied Biosystems, Inc., with p-methylbenzylhydrylamine resin (2% divinyl benzene / Peptides International) as the solid support. The symmetrical anhydride of Tyr, bearing Boc as the α-amino protecting group and 2Br-Z as the side-chain hydroxy protecting group, is formed by reacting the protected Tyr with N,N-dicyclohexylcarbodiimide in dichloromethane. The dichloromethane is exchanged for DMF. The protected Tyr is coupled to the resin by introducing the symmetrical anhydride solution into the solid phase reactor containing the resin and mixing until the reaction is complete. The anhydride should be present in about a 2-fold excess. The α-amino protecting group of the protected Tyr is removed by reacting the resin-bound Tyr derivative with trifluoroacetic acid. Arg, bearing Boc as the α-amino protecting group and p-toluenesulfonyl (tosyl) as the side-chain amino protecting group, is coupled to the resin-bound Tyr derivative by reacting the protected Arg with N,N-dicyclohexylcarbodiimide / 1-hydroxybenzotriazole (1:1) in the presence of the resin-bound Tyr derivative in DMF. In like manner, the following amino acids, all bearing Boc as the α-amino protecting group, are coupled to the resin-bound peptide in sequence following the same procedures as described above of deprotection of the α-amino protecting group of the peptide N-terminus followed by coupling:

Aoa ; Cys, bearing p-methyl benzyl as the side-chain sulfide protecting group; Gly; Leu; Gly; Ser, bearing Bzl ether as the side-chain hydroxy protecting group; Gln; Ala; Gly; Ile; Arg, bearing tosyl as the side-chain amino protecting group; Asp, bearing cyclohexyl ester as the side-chain carboxylic acid protecting group; Ile; Arg, bearing tosyl as the side-chain amino protecting group; Gly; Gly; Phe; Cys, bearing p-methyl benzyl as the side-chain sulfide protecting group; Ser, bearing Bzl ether as the side-chain hydroxy protecting group; Ser, bearing Bzl ether as the side-chain hydroxy protecting group.

Dicyclohexylcarbodiimide / 1-hydroxybenzotriazole (1:1) in DMF is used as the coupling reagent to couple Arg, Asn and Gln derivatives to the resin-bound peptide. N,N-dicyclohexylcarbodiimide in dichloromethane is used to form the symmetrical anhydrides of all other amino acid derivatives.

The peptide is cleaved from the resin and the side-chain protecting groups are removed by reacting the resin-bound peptide with a solution of anisole in anhydrous hydrogen fluoride (1:10). The disulfide bridge is formed by oxidizing the unprotected peptide with potassium ferricyanide. The peptide is purified by gel filtration chromatography, ion exchange chromatography, dialysis, and reverse phase high performance liquid chromatography (HPLC).

Fast Atom Bombardment Mass Spectrometry (FAB-MS): $(M+H)^+ = 2341$;

Amino acid analysis (%): Asp (0.73); Glu (0.91); Ser (2.78); Gly (5.15); Ala (1.01); Arg (3.01); Tyr (0.98); Ile (1.95); Leu (0.97); Phe (0.93); Aoa (1.01).

In a similar manner, the following peptides can be prepared:

$[Aoa^{8-10}]rANF_{5-7,11-28}NH_2$ :

FAB-MS : $(M+H)^+ = 2427$;Amino acid analysis (%): Asp (1.24); Glu (0.88); Ser (3.72); Gly (3.16); Ala (1.07);

Arg (3.01); Tyr (0.95); Ile (1.95); Leu (0.94); Phe (0.98); Aoa (1.07).

$[Aoa^{17-19}]rANF_{5\text{-}16,20\text{-}28}\text{-}NH_2$:

FAB-MS : $(M+H)^+$ = 2404;Amino acid analysis (%): Asp (1.31); Ser (2.88); Gly (5.18); Arg (2.95); Tyr (1.00); Ile (2.03); Leu (0.99); Phe (1.94); Aoa (0.70).

$[Aoa^{20-22}]rANF_{5\text{-}19,23\text{-}28}\text{-}NH_2$:

FAB-MS : $(M+H)^+$ = 2462;Amino acid analysis (%): Asp (0.98); Ser (3.77); Asn (0.63); Gly (2.91); Glu (0.85); Arg (2.99); Tyr (1.02); Ile (2.33); Phe (2.20); Aoa (0.97).

$[Aoa^{15-17}]rANF_{5\text{-}14,18\text{-}28}\text{-}NH_2$ :

FAB-MS : $(M+H)^+$ = 2447;Amino acid analysis (%): Asp (1.77); Glu (0.96); Ser (3.62); Gly (4.21); Arg (2.91); Tyr (0.98); Ile (0.77); Leu (1.01); Phe (2.11); Cys (1.96);Aoa (0.80).

$[Aoa^{16-18}]rANF_{5\text{-}15,19\text{-}28}\text{-}NH_2$:

FAB-MS : $(M+H)^+$ = 2433;Amino acid analysis (%): Asp (1.97); Ser (4.08); Gly (4.45); Arg (2.96); Tyr (0.95); Ile (1.24); Leu (0.96); Phe (1.92); Cys (1.46);Aoa (0.26).

$[Aoa^{8\text{-}10,17\text{-}19,20\text{-}22,24\text{-}26}]rANF_{5\text{-}7,11\text{-}16,23,27\text{-}28}\text{-}NH_2$:

FAB-MS : $(M+H)^+$ = 1988;Amino acid analysis (%): Asp (0.81); Ser (1.82); Gly (1.03); Arg (3.00); Tyr (0.96); Ile (1.74); Cys (1.94);Aoa (3.07).

In another embodiment of the present invention, a method is provided for the treatment of patients afflicted with disease states characterized by abnormalities in fluid, electrolyte, blood pressure, intraocular pressure, renin, or aldosterone homeostasis such as, but not limited to, hypertension, renal disease, hyperaldosteronemia, cardiac hypertrophy, glaucoma, and congestive heart failure. This method of treatment comprises administering to said patient a therapeutically effective amount of a compound of the formula 1, or pharmaceutically acceptable non-toxic salts thereof. As used herein, the term "patient" refers to a warm blooded animal such as a mammal which is afflicted with a disease state defined above. It is understood that dogs, cats, rats, mice, horses, bovine cattle, sheep, and humans are examples of animals within the scope of the meaning of the term. A therapeutically effective amount is an amount which, upon single or multiple dose administration, produces a desired therapeutic effect by significantly altering abnormalities in fluid, electrolyte, renin, aldosterone, intraocular pressure, or blood pressure parameters in patients so afflicted, and returning one or more of these parameters toward more normal values.

In effecting treatment of a patient afflicted with a disease state described above, a compound of formula 1 can be administered in any manner which makes the compound bioavailable in effective amounts, including by oral and parenteral routes. For example, compounds of the formula 1 can be administered orally, subcutaneously, intramuscularly, intravenously, transdermally, intranasally, rectally, and the like. Parenteral administration is generally preferred.

A therapeutically effective amount of a compound of the formula 1 will vary from about 0.5 microgram per kilogram of body weight per day (µg/kg/day) to about 50 milligram per kilogram of body weight per day (mg/kg/day). It is understood that a therapeutically effective dose is a function of a number of factors including, but not limited to: the species of mammal; its size, age, and general health; the specific disease state involved; the degree or of involvement or the severity of the disease state; the response of the individual patient; the particular compound administered; the mode of administration; the bioavailability characteristics of the preparation administered; the dose regimen selected; the use of concomitant medication; and other relevant circumstances. The correct amount for any specific situation can be readily determined by those skilled in the art using conventional range finding techniques and analogous results observed under other circumstances. Therefore, the above dosage ranges are illustrative only and are not intended to limit the scope of the invention in any way.

As a further embodiment, the present invention provides pharmaceutical compositions for administration of compounds of the formula 1 to patients.These compositions comprise a therapeutically effective amount of one or more compounds of formula 1 associated with one or more pharmaceutically acceptable carriers. Such compositions are prepared using conventional processes and methods which are well known in the art of pharmaceutical science. In making the compositions of the present invention, a compound of formula 1 will usually be mixed with a carrier, or diluted with a carrier, or enclosed within a carrier which may be in the form of a capsule, sachet, or other container. When the compound of formula 1 is mixed with, or diluted by, one or more carriers, such carriers may be solid, semi-solid, or liquid material which acts as a vehicle, excipient, or medium for the compound of formula 1 Thus the composition can be in the form of tablets, powders, lozenges, sachets, cachets, elixers, emulsions, solutions, syrups, gels, aerosols (in a solid or a liquid medium), ointments, soft and hard gelatin capsules, suppositories, sterile injectable solutions, and sterile packaged powders.

Some examples of suitable carriers, excipients, and diluents include lactose, dextrose, sucrose, sorbitol, mannitol, starches, gum acacia, calcium phosphate, alginates, calcium silicate, microcrystalline cellulose,

polyvinylpyrollidine, cellulose, tragacanth, gelatin, syrup, methyl cellulose, methyl- and propylhydroxybenzoates, talc, magnesium stearate, water, and mineral oil. The formulations can additionally include lubricating agents, wetting agents, emulsifying and suspending agents, preserving agents, sweetening agents, flavoring agents, or coloring agents. The compositions of the invention may be formulated so as to provide immediate, sustained, or delayed release of the compound of formula 1 after administration to the patient by utilization of standard methods and practices well known in the art.

For oral administration, a compound of formula 1 can be admixed with carriers and diluents molded into tablets or enclosed in gelatin capsules. The mixtures can alternatively be dissolved in liquids such as ten percent aqueous glucose solution, isotonic saline, sterile water, and the like,and administered intravenously or by injection. Such solutions can, if desired, be lyophilized and stored in a sterile ampule ready for reconstitution by the addition of sterile water for ready intramuscular injection.

The compositions are preferably formulated in a unit dosage form, each dosage containing from about 5 µg to about 1000 mg , more usually from about 10 µg to about 100 mg of one or more compounds of formula 1 in association with a suitable pharmaceutical carrier. The term "unit dosage form" refers to physically discrete units suitable as unitary dosages for human and other patients. Each unit contains a predetermined quantity of one or more compounds of formula 1 calculated to provide a therapeutically effective amount when administered as a single dose or as part of a multiple dose regimen.

The naturally-occurring peptide $rANF_{1-28}$ and fragments thereof have been shown using*in vitro* methods to bind to ANF-specific binding sites on membranes in a variety of tissues including vascular smooth muscle cells, kidney cortex cells, adrenal zona glomerulosa cells, pulmonary tissue, and brain tissue. It is generally believed that binding to such sites may at least in part reflect a mechanism by which ANF exerts its biological activity*in vivo*. Compounds of the formula 1 are also believed to bind to ANF-specific binding sites. The*in vitro* binding affinity for compounds of the formula 1 for ANF-specific binding sites in a particular tissue can be determined through standard receptor binding assays utilizing standard practices and procedures as are well known and appreciated in the art, such as the method of Napier et al. (Proc. Nat. Acad. Sci. USA, 81 : 5946-50, 1984) and Buck et al. (Science, 226 : 987-89, 1984). For example, tissue homogenates can be incubated with varying concentrations of a compound of formula 1 in the presence of an amount of iodinated ($^{125}I$) $rANF_{5-28}$ which is approximately equal to the dissociation constant ($K_D$) for high-affinity, specific ANF binding sites.The amount of displacement of the $^{125}I$-$rANF_{5-28}$ from the ANF-specific binding sites by the compound of formula 1 at the various concentrations can be determined by measuring the amount of $^{125}I$ which remains bound to the tissue at equilibrium. These data can be used to calculate a $K_I$ for the compound of the formula 1 for the particular tissue homogenate being tested. In general, the $K_I$ is inversely proportional to the affinity of the tested compound for the binding site. Compounds of the formula 1 which have a $K_I$ of less than about 500 nM for any ANF-specific receptor are generally preferred.Those compounds of formula 1 which have a $K_I$ of less than about 50 nM for any ANF-specific receptor are especially preferred. Of course it is understood that the results of receptor binding assays are dependent on a variety of factors including the temperature of the assay system, the pH used, the solubility of the test compound in the assay system, the incubation time, the concentration of $^{125}I$-ANF used, the naturally-occurring ANF peptide used, the type of assay, the tissue source, and the like. Generally, assay parameters which are consistent with the standard practices and procedures as are well known and appreciated in the art are preferred.

## Claims

1. A compound of the formula

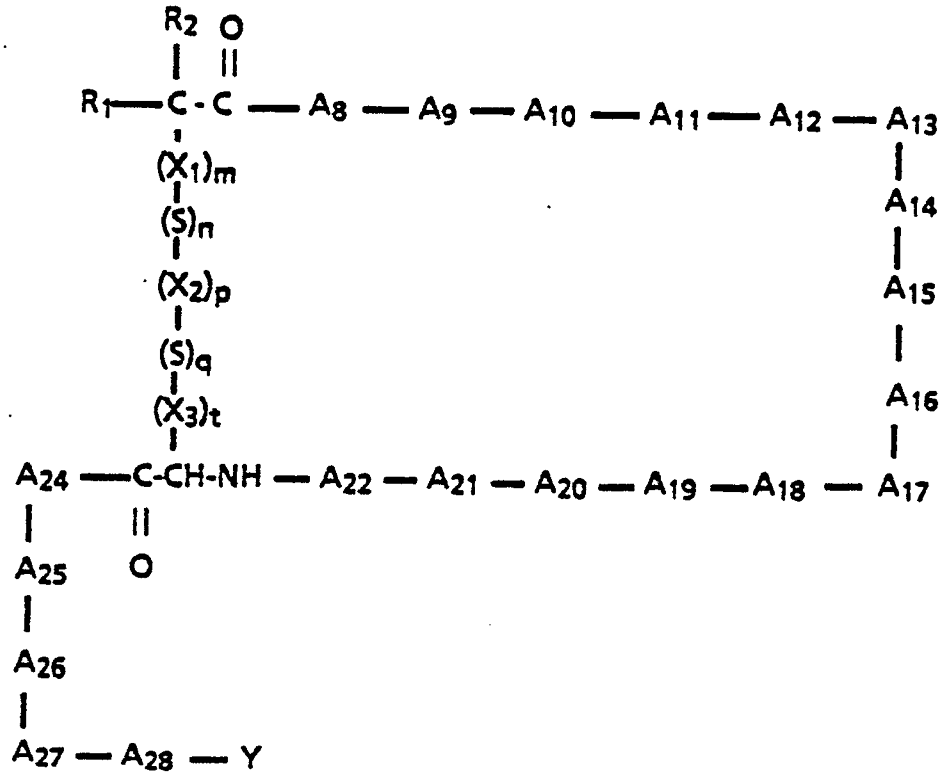

wherein
$R_1$ is hydrogen, $C_{1-6}$ alkyl, or a group of the formula

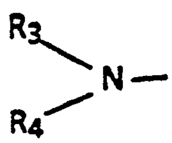 or $R_3—R_5—NH—$

wherein
$R_3$ and $R_4$ are each independently hydrogen or an amino protecting group;
$R_5$ is a polypeptide of 1 to 10 amino acid residues,

$R_2$ is hydrogen or $C_{1-6}$ alkyl with the provision that when $R_1$ and $R_2$ are each alkyl they can be independent chains or they can be taken together with the carbon atom to which they are attached to form a ($C_3$-$C_{13}$)cyclic alkyl;

$X_1$, $X_2$, and $X_3$ are each independently $C_{1-8}$ alkylene groups;

m, n, p, q, and t are each independently integers 0 or 1;

$A_8$ is a bond, Phe, N-substituted $C_{1-4}$ alkyl Phe, alpha-C substituted $C_{1-4}$ alkyl Phe, or D-Phe;

$A_9$ is a bond, Gly or Ala, N-substituted $C_{1-4}$ alkyl--Gly or -Ala, alpha-C substituted $C_{1-4}$ alkyl -Gly or -Ala, or D-Ala;

$A_{10}$ is a bond, Gly or Ala, N-substituted $C_{1-4}$ alkyl-Gly or -Ala, alpha-C substituted $C_{1-4}$ alkyl -Gly or -Ala, or D-Ala;

$A_{11}$ is a bond, Arg, N-substituted $C_{1-4}$ alkyl Arg, alpha-C substituted $C_{1-4}$ alkyl Arg, or D-Arg;

$A_{12}$ is a bond, Ile, Nle, or Met; N-substituted $C_{1-4}$alkyl -Ile, -Nle, or -Met; alpha-C-substituted $C_{1-4}$-alkyl -Ile, -Nle, or -Met; or D-Ile, D-Nle, or D-Met;

$A_{13}$ is a bond, Asp or Glu, N-substituted $C_{1-4}$ alkyl-Asp or -Glu, alpha-C-substituted $C_{1-4}$ alkyl -Asp or -Glu, or D-Asp or D-Glu;

$A_{14}$ is a bond, Arg, N-substituted $C_{1-4}$ alkyl Arg, alpha-C-substituted $C_{1-4}$ alkyl Arg, or D-Arg;

$A_{15}$ is a bond, Ile, Nle, or Met, N-substituted $C_{1-4}$-alkyl -Ile, -Nle, or -Met; alpha-C-substituted $C_{1-4}$-alkyl -Ile, -Nle, or -Met, or D-Ile, D-Nle, or D-Met;

$A_{16}$ is a bond, Gly or Ala, N-substituted $C_{1-4}$ alkyl-Gly or -Ala, alpha-C substituted $C_{1-4}$ alkyl -Gly or -Ala, or D-Ala;

$A_{17}$ is a bond, Gly or Ala, N-substituted $C_{1-4}$ alkyl-Gly or -Ala, alpha-C substituted $C_{1-4}$ alkyl -Gly or -Ala, or D-Ala;

$A_{18}$ is a bond, Gln or Asn, N-substituted $C_{1-4}$ alkyl-Gln or -Asn, alpha-C substituted $C_{1-4}$ alkyl -Gln or -Asn, or D-Gln or D-Asn;

$A_{19}$ is a bond, Ser or Thr, N-substituted $C_{1-4}$ alkyl-Ser or -Thr, alpha-C substituted $C_{1-4}$ alkyl -Ser or -Thr, or D-Ser or D-Thr;

$A_{20}$ is a bond, Gly or Ala, N-substituted $C_{1-4}$ alkyl-Gly or -Ala, alpha-C substituted $C_{1-4}$ alkyl -Gly or -Ala, or D-Ala;

$A_{21}$ is a bond, Leu or Nle, N-substituted $C_{1-4}$ alkyl-Leu or -Nle, alpha-C substituted $C_{1-4}$ alkyl -Leu or -Nle, or D-Leu or D-Nle;

$A_{22}$ is a bond, Gly or Ala, N-substituted $C_{1-4}$ alkyl-Gly or -Ala, alpha-C substituted $C_{1-4}$ alkyl -Gly or -Ala, or D-Ala;

$A_{24}$ is a bond, Asn or Gln, N-substituted $C_{1-4}$ alkyl-Asn or Gln, alpha-C substituted $C_{1-4}$ alkyl Asn or Gln, or D-Asn or D-Gln;

$A_{25}$ is a bond, Ser, N-substituted $C_{1-4}$ alkyl Ser, alpha-C substituted $C_{1-4}$ alkyl Ser, or D-Ser;

$A_{26}$ is a bond, Phe, N-substituted $C_{1-4}$ alkyl Phe, alpha-C substituted $C_{1-4}$ alkyl Phe, or D-Phe;

$A_{27}$ is a bond, Arg, N-substituted $C_{1-4}$ alkyl Arg, alpha-C substituted $C_{1-4}$ alkyl Arg, or D-Arg;

$A_{28}$ is a bond, Tyr, N-substituted $C_{1-4}$ alkyl Tyr, alpha-C substituted $C_{1-4}$ alkyl Tyr, or D-Tyr;

Y is -OH, $C_{1-6}$ alkoxy, amino, mono- or di-$C_{1-4}$ alkyl substituted amino;

and wherein one or more amino acid residues, or derivatives thereof, designated as "A" above, are replaced by one or more divalent groups of the formula

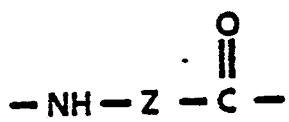

wherein Z is a $C_{2-24}$ alkylene;

with the provision that no more than 5 amino acid residues, or derivatives thereof, designated as "A" above, are bonds, and that the compounds of formula (1) are characterized by a binding constant for ANF receptors of less than about 500 nM; or pharmaceutically acceptable non-toxic salts thereof.

2. A compound of the formula

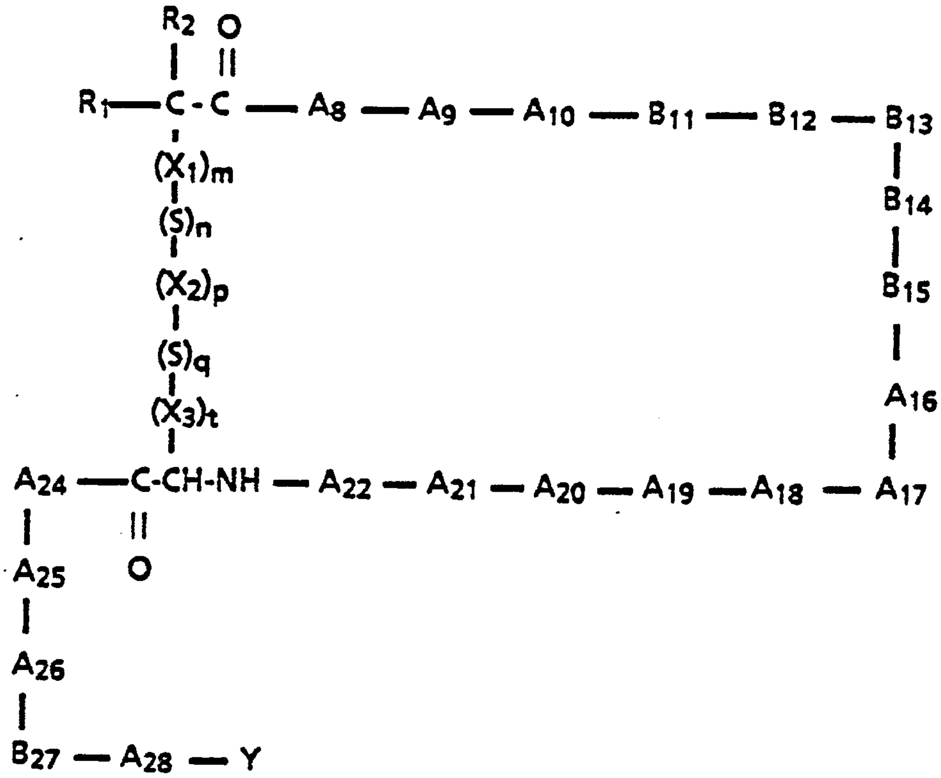

wherein

$R_1$ is hydrogen, $C_{1-6}$ alkyl, or a group of the formula

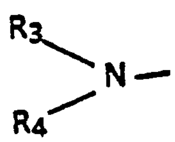

or $R_3-R_5-NH-$

wherein

$R_3$ and $R_4$ are each independently hydrogen or an amino protecting group;

$R_5$ is a polypeptide of 1 to 10 amino acid residues,

$R_2$ is hydrogen or $C_{1-6}$ alkyl with the provision that when $R_1$ and $R_2$ are each alkyl they can be independent chains or they can be taken together with the carbon atom to which they are attached to form a cyclic alkyl;

$X_1$, $X_2$, and $X_3$ are each independently $C_{1-8}$ alkylene groups;

m, n, p, q, and t are each independently integers 0 or 1;

$A_8$ is a bond, Phe, N-substituted $C_{1-4}$ alkyl Phe, alpha-C substituted $C_{1-4}$ alkyl Phe, or D-Phe;

$A_9$ is a bond, Gly or Ala, N-substituted $C_{1-4}$ alkyl-Gly or -Ala, alpha-C substituted $C_{1-4}$ alkyl -Gly or -Ala, or D-Ala;

$A_{10}$ is a bond, Gly or Ala, N-substituted $C_{1-4}$ alkyl-Gly or -Ala, alpha-C substituted $C_{1-4}$ alkyl -Gly or -Ala, or D-Ala;

$B_{11}$ is a bond, Arg, N-substituted $C_{1-4}$ alkyl Arg, alpha-C substituted $C_{1-4}$ alkyl Arg, or D-Arg;

$B_{12}$ is a bond, Ile, Nle, or Met; N-substituted $C_{1-4}$alkyl -Ile, -Nle, or -Met; alpha-C-substituted $C_{1-4}$-alkyl -Ile, -Nle, or -Met; or D-Ile, D-Nle, or D-Met;

$B_{13}$ is a bond, Asp or Glu, N-substituted $C_{1-4}$ alkyl-Asp or -Glu, alpha-C-substituted $C_{1-4}$ alkyl -Asp or -Glu, or D-Asp or D-Glu;

$B_{14}$ is a bond, Arg, N-substituted $C_{1-4}$ alkyl Arg, alpha-C-substituted $C_{1-4}$ alkyl Arg, or D-Arg;

$B_{15}$ is a bond, Ile, Nle, or Met, N-substituted $C_{1-4}$-alkyl -Ile, -Nle, or -Met; alpha-C-substituted $C_{1-4}$-alkyl -Ile, -Nle, or -Met, or D-Ile, D-Nle, or D-Met;

$A_{16}$ is a bond, Gly or Ala, N-substituted $C_{1-4}$ alkyl-Gly or -Ala, alpha-C substituted $C_{1-4}$ alkyl -Gly or -Ala, or D-Ala;

$A_{17}$ is a bond, Gly or Ala, N-substituted $C_{1-4}$ alkyl-Gly or -Ala, alpha-C substituted $C_{1-4}$ alkyl -Gly or -Ala, or D-Ala;

$A_{18}$ is a bond, Gln or Asn, N-substituted $C_{1-4}$ alkyl-Gln or -Asn, alpha-C substituted $C_{1-4}$ alkyl -Gln or -Asn, or D-Gln or D-Asn;

$A_{19}$ is a bond, Ser or Thr, N-substituted $C_{1-4}$ alkyl-Ser or -Thr, alpha-C substituted $C_{1-4}$ alkyl -Ser or -Thr, or D-Ser or D-Thr;

$A_{20}$ is a bond, Gly or Ala, N-substituted $C_{1-4}$ alkyl-Gly or -Ala, alpha-C substituted $C_{1-4}$ alkyl -Gly or -Ala, or D-Ala;

$A_{21}$ is a bond, Leu or Nle, N-substituted $C_{1-4}$ alkyl-Leu or -Nle, alpha-C substituted $C_{1-4}$ alkyl -Leu or -Nle, or D-Leu or D-Nle;

$A_{22}$ is a bond, Gly or Ala, N-substituted $C_{1-4}$ alkyl-Gly or -Ala, alpha-C substituted $C_{1-4}$ alkyl -Gly or -Ala, or D-Ala;

$A_{24}$ is a bond, Asn or Gln, N-substituted $C_{1-4}$ alkyl-Asn or Gln, alpha-C substituted $C_{1-4}$ alkyl Asn or Gln, or D-Asn or D-Gln;

$A_{25}$ is a bond, Ser, N-substituted $C_{1-4}$ alkyl Ser, alpha-C substituted $C_{1-4}$ alkyl Ser, or D-Ser;

$A_{26}$ is a bond, Phe, N-substituted $C_{1-4}$ alkyl Phe, alpha-C substituted $C_{1-4}$ alkyl Phe, or D-Phe;

$A_{27}$ is a bond, Arg, N-substituted $C_{1-4}$ alkyl Arg, alpha-C substituted $C_{1-4}$ alkyl Arg, or D-Arg;

$A_{28}$ is a bond, Tyr, N-substituted $C_{1-4}$ alkyl Tyr, alpha-C substituted $C_{1-4}$ alkyl Tyr, or D-Tyr;

Y is -OH, $C_{1-6}$ alkoxy, amino, mono- or di-$C_{1-4}$-alkyl substituted amino;

and wherein one or more amino acid residues, or derivatives thereof, designated as "A" above, are replaced by one or more divalent groups of the formula

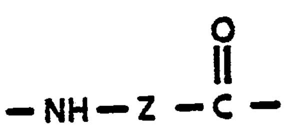

wherein Z is a $C_{2-24}$ alkylene;

with the provision that no more than 5 amino acid residues, or derivatives thereof, designated as "A" above, are bonds; or pharmaceutically acceptable non-toxic salts thereof.

3. A compound of claim 1 or 2 wherein $R_5$ is Ser, Ser-Ser, Arg-Ser-Ser, Arg-Arg-Ser-Ser, Leu-Arg-Arg-Ser-Ser, or Ser-Leu-Arg-Arg-Ser-Ser.

4. A compound of claim 1 or 2 wherein $X_1$ and $X_3$ are methylene; m, n, q, and t are 1; and p is 0.

5. A compound of claim 1 or 2 wherein $X_1$ and $X_3$ are methylene; $X_2$ is ethylene; m, p, and t are 1; and n and q are 0.

6. A compound of claim 1 or 2 wherein $X_1$ is a cyclopentylene; $X_3$ is methylene; m, n, q, and t are 1; and p is 0.

7. A compound of claim 1 or 2 wherein $X_1$ is a cyclohexylene; $X_3$ is methylene; m, n, q, and t are 1; and p is 0.

8. A compound of claim 1 or 2 wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{17}$ is Ala, $A_{18}$ is Gln, $A_{19}$ is Ser, $A_{20}$ is Gly, $A_{21}$ is Leu, $A_{22}$ is Gly, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 24 through 26, and Y is $-NH_2$.

9. A compound of claim 1 or 2 wherein $A_{11}$ is Arg, $A_{12}$ is Ile, $A_{13}$ is Asp, $A_{14}$ is Arg, $A_{15}$ is Ile, $A_{16}$ is Gly, $A_{17}$ is Ala, $A_{18}$ is Gln, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$-is $-CH_2$-S-S-$CH_2$-, the divalent residue of aminooctanoic acid is a spacer group occupying positions 8 through 10, and Y is $-NH_2$.

10. A compound of claim 1 or 2 wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$-is $-CH_2$-S-S-$CH_2$-, the divalent residue of aminooctanoic acid is a spacer group occupying positions 17 through 19, and Y is $-NH_2$.

11. A compound of claim 1 or 2 wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{17}$ is Ala, $A_{18}$ is Gln, $A_{19}$ is Ser, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 20 through 22, and Y is $-NH_2$.

12. A compound of claim 1 or 2 wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{18}$ is Gln, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is -$CH_2$-S-S-$CH_2$-, the divalent residue of aminooctanoic acid is a spacer group occupying positions 15 through 17, and Y is -$NH_2$.

13. A compound of claim 1 or 2 wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is - $CH_2$-S-S-$CH_2$-, the divalent residue of aminooctanoic acid is a spacer group occupying positions 16 through 18, and Y is -$NH_2$.

14. A compound of claim 1 or 2 wherein $A_8$ is Phe, $A_9$ is Gly, $A_{10}$ is Gly, $A_{14}$ is Arg, $A_{15}$ is Ile, $A_{16}$ is Gly, $A_{17}$ is Ala, $A_{18}$ is Gln, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is -$CH_2$-S-S-$CH_2$-, the divalent residue of aminooctanoic acid is a spacer group occupying positions 11 through 13, and Y is -$NH_2$.

15. A compound of claim 1 or 2 wherein $A_8$ is Phe, $A_9$ is Gly, $A_{10}$ is Gly, $A_{11}$ is Arg, $A_{12}$ is Ile, $A_{13}$ is Asp, $A_{17}$ is Ala, $A_{18}$ is Gln, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is -$CH_2$-S-S-$CH_2$-, the divalent residue of aminooctanoic acid is a spacer group occupying positions 14 through 16, and Y is -$NH_2$.

16. A compound of claim 1 or 2 wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is -$CH_2$-S-S-$CH_2$-, two divalent residues of aminooctanoic acid are spacer groups occupying positions 17 through 19 and 20 through 22, and Y is -$NH_2$.

17. A compound of claim 1 or 2 wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{17}$ is Ala, $A_{18}$ is Gln, $A_{19}$ is Ser, $A_{20}$ is Gly, $A_{21}$ is Leu, $A_{22}$ is Gly, $A_{24}$ is Asn, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is -$CH_2$-S-S-$CH_2$-, the divalent residue of aminooctanoic acid is a spacer group occupying positions 25 through 27, and Y is -$NH_2$.

18. A compound of claim 1 or 2 wherein $A_8$ is Phe, $A_{11}$ is Arg, $A_{12}$ is N-Me-Ile, $A_{13}$ is Asp, $A_{14}$ is Arg, $A_{15}$ is N-Me-Ile, $A_{27}$ is D-Arg, $R_1$ is H, $R_2$ is H, $(X_1)_m$-$(S)_n$-$(X_2)_p$-$(S)_q$-$(X_3)_t$- is -$CH_2$-S-S-$CH_2$-, the divalent residue of aminopentanoic acid is a spacer group occupying positions 9 and 10, the divalent residue of aminoundecanoic acid is a spacer group occupying positions 16 through 19, two divalent residues of aminooctanoic acid are spacer groups occupying positions 20 through 22 and 24 through 26, and Y is -$NH_2$.

19. A compound of claim 1 or 2 wherein $A_8$ is Phe, $A_9$ is Gly, $A_{10}$ is Gly, $A_{11}$ is Arg, $A_{12}$ is N-Me-Ile, $A_{13}$ is Asp, $A_{14}$ is Arg, $A_{15}$ is N-Me-Ile, $A_{27}$ is D-Arg, $A_{28}$ is Tyr, $R_1$ is H, $R_2$ is H, $(X_1)_m$-$(S)_n$-$(X_2)_p$-$(S)_q$-$(X_3)_t$- is -$CH_2$-S-S-$CH_2$-, the divalent residue of aminoundecanoic acid is a spacer group occupying positions 16 through 19, two divalent residues of aminooctanoic acid are spacer groups occupying positions 20 through 22 and 24 through 26, and Y is -$NH_2$.

20. A compound of claim 1 or 2 wherein $A_8$ is N-Me-Phe, $A_9$ is Gly, $A_{10}$ is Gly, $A_{11}$ is Arg, $A_{12}$ is N-Me-Ile, $A_{13}$ is Asp, $A_{14}$ is Arg, $A_{15}$ is N-Me-Ile, $A_{27}$ is D-Arg, $A_{28}$ is Tyr, $R_1$ is H-Ser-Ser-NH, $R_2$ is H, $(X_1)_m$-$(S)_n$-$(X_2)_p$-$(S)_q$-$(X_3)_t$- is -$CH_2$-S-S-$CH_2$-, the divalent residue of aminoundecanoic acid is a spacer group occupying positions 16 through 19, two divalent residues of aminooctanoic acid are spacer groups occupying positions 20 through 22 and 24 through 26, and Y is -$NH_2$.

21. A compound of claim 1 or 2 wherein $A_{11}$ is Arg, $A_{12}$ is Ile, $A_{13}$ is Asp, $A_{14}$ is Arg, $A_{15}$ is Ile, $A_{16}$ is Gly, $A_{27}$ is Arg, $A_{28}$ is Tyr, $R_1$ is H-Ser-Ser-NH, $R_2$ is H, $(X_1)_m$-$(S)_n$-$(X_2)_p$-$(S)_q$-$(X_3)_t$- is -$CH_2$-S-S-$CH_2$-, four divalent residues of aminooctanoic acid are spacer groups occupying positions 8 through 10, 17 through 19, 20 through 22, and 24 through 26, and Y is -$NH_2$.

22. A compound of any one of the preceding claims for use as a medicine.

23. A pharmaceutical composition comprising a compound of any one of the preceding claims in admixture with a pharmaceutically acceptable carrier.

24. A parenteral pharmaceutical composition comprising any of the compound of claims 1 to 21 in admixture with a parenterally acceptable pharmaceutical vehicle.

25. A pharmaceutical composition according to claim 23 or 24 wherein the unit dosage form contains from 10 microgram to 100 milligram of active compound.

26. Use of a compound of any one of the preceding claims for preparing a medicament for treating abnormalities in fluid, electrolyte, blood pressure, intraocular pressure, renin, or aldosterone homeostasis.

27. Use according to claim 26 wherein the medicament is used for treating hypertension, renal diseases, hyperaldosteronemia, cardiac hypertrophy, glaucoma, and congestive heart failure.

Claims for the following Contracting States: ES, GR

1. A process for preparing a compound of the formula

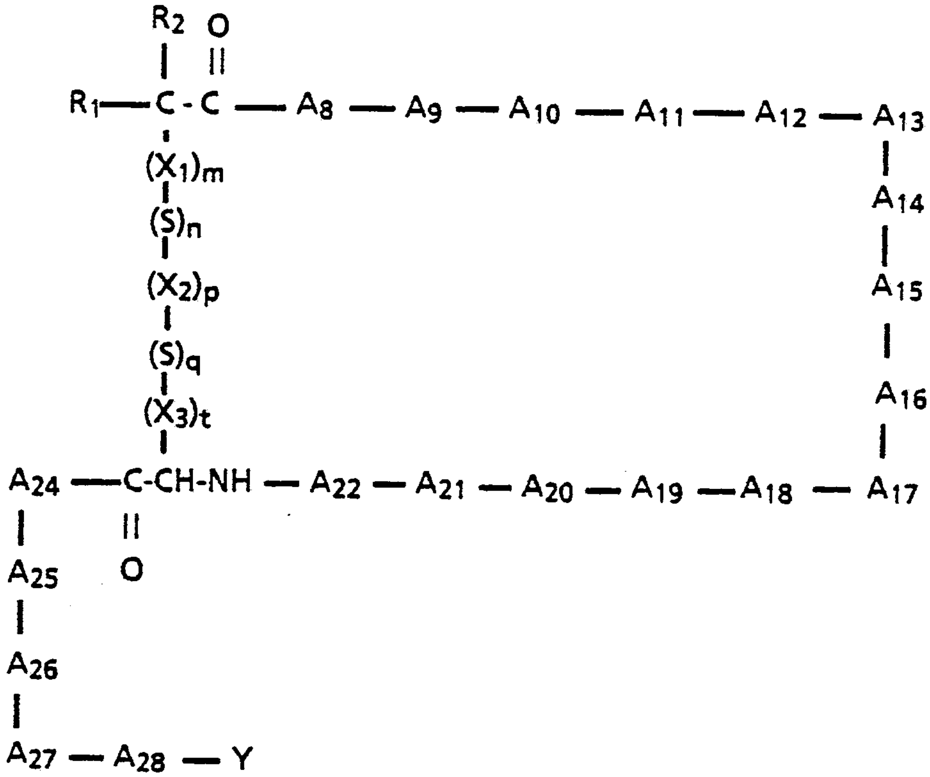

wherein,

$R_1$ is hydrogen, $C_{1-6}$ alkyl, or a radical of the formula

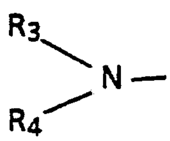

or

$R_3 - R_5 - NH -$

wherein

$R_3$ and $R_4$ are each independently hydrogen or an amino protecting group;

$R_5$ is a polypeptide of 1 to 10 amino acid residues,

$R_2$ is hydrogen or $C_{1-6}$ alkyl with the provision that when $R_1$ and $R_2$ are each alkyl they can be independent chains or they can be taken together with the carbon atom to which they are attached to form a ($C_3$-$C_{13}$)cyclic alkyl;

$X_1$, $X_2$, and $X_3$ are each independently $C_{1-8}$ alkylene radicals;

m, n, p, q, and t are each independently integers 0 or 1;

$A_8$ is a bond, Phe, N-substituted $C_{1-4}$ alkyl Phe, α-C substituted $C_{1-4}$ alkyl Phe, or D-Phe

$A_9$ is a bond, Gly or Ala, N-substituted $C_{1-4}$ alkyl -Gly or -Ala, α-C substituted $C_{1-4}$ alkyl -Gly or -Ala, or D-Ala;

$A_{10}$ is a bond, Gly or Ala, N-substituted $C_{1-4}$ alkyl -Gly or -Ala, α-C substituted $C_{1-4}$ alkyl -Gly or -Ala, or D-Ala;

$A_{11}$ is a bond, Arg, N-substituted $C_{1-4}$ alkyl-Arg, α-C-substituted $C_{1-4}$ alkyl Arg, or D-Arg;

$A_{12}$ is a bond, Ile, Nle, or Met; N-substituted $C_{1-4}$ alkyl -Ile, -Nle, or -Met; α-C-substituted $C_{1-4}$ alkyl -Ile, -Nle, or -Met; or D-Ile, D-Nle, or D-Met;

$A_{13}$ is a bond, Asp or Glu, N-substituted $C_{1-4}$ alkyl -Asp or -Glu, α-C-substituted $C_{1-4}$ alkyl -Asp or -Glu, or D-Asp or D-Glu;

$A_{14}$ is a bond, Arg, N-substituted $C_{1-4}$ alkyl Arg, $\alpha$-C-substituted $C_{1-4}$ alkyl Arg, or D-Arg;
$A_{15}$ is a bond, Ile, Nle, or Met, N-substituted $C_{1-4}$ alkyl -Ile, -Nle, or -Met, $\alpha$-C-substituted $C_{1-4}$ alkyl -Ile, -Nle, or -Met, or D-Ile, D-Nle, or D-Met;
$A_{16}$ is a bond, Gly or Ala, N-substituted $C_{1-4}$ alkyl -Gly or -Ala, $\alpha$-C substituted $C_{1-4}$ alkyl -Gly or -Ala, or D-Ala;
$A_{17}$ is a bond, Gly or Ala, N-substituted $C_{1-4}$ alkyl -Gly or -Ala, $\alpha$-C substituted $C_{1-4}$ alkyl -Gly or -Ala, or D-Ala;
$A_{18}$ is a bond, Gln or Asn, N-substituted $C_{1-4}$ alkyl -Gln or -Asn, $\alpha$-C substituted $C_{1-4}$ alkyl -Gln or -Asn, or D-Gln or D-Asn;
$A_{19}$ is a bond, Ser or Thr, N-substituted $C_{1-4}$ alkyl -Ser or -Thr, $\alpha$-C substituted $C_{1-4}$ alkyl -Ser or -Thr, or D-Ser or D-Thr;
$A_{20}$ is a bond, Gly or Ala, N-substituted $C_{1-4}$ alkyl -Gly or -Ala, $\alpha$-C substituted $C_{1-4}$ alkyl -Gly or -Ala, or D-Ala;
$A_{21}$ is a bond, Leu or Nle, N-substituted $C_{1-4}$ alkyl -Leu or -Nle, $\alpha$-C substituted $C_{1-4}$ alkyl -Leu or -Nle, or D-Leu or D-Nle;
$A_{22}$ is a bond, Gly or Ala, N-substituted $C_{1-4}$ alkyl -Gly or -Ala, $\alpha$-C substituted $C_{1-4}$ alkyl -Gly or -Ala, or D-Ala;
$A_{24}$ is a bond, Asn or Gln, N-substituted $C_{1-4}$ alkyl Asn or Gln, $\alpha$-C-substituted $C_{1-4}$ alkyl Asn or Gln, or D-Asn or D-Gln;
$A_{25}$ is a bond, Ser, N-substituted $C_{1-4}$ alkyl Ser, $\alpha$-C-substituted $C_{1-4}$ alkyl Ser, or D-Ser;
$A_{26}$ is a bond, Phe, N-substituted $C_{1-4}$ alkyl Phe, $\alpha$-C-substituted $C_{1-4}$ alkyl Phe, or D-Phe;
$A_{27}$ is a bond, Arg, N-substituted $C_{1-4}$ alkyl Arg, $\alpha$-C-substituted $C_{1-4}$ alkyl Arg, or D-Arg;
$A_{28}$ is a bond, Tyr, N-substituted $C_{1-4}$ alkyl Tyr, $\alpha$-C-substituted $C_{1-4}$ alkyl Tyr, or D-Tyr;
Y is -OH, $C_{1-6}$ alkoxy, amino, mono- or di- $C_{1-4}$ alkyl substituted amino;
and wherein one or more amino acid residues,or derivatives thereof, designated as "A" above, are replaced by one or more divalent radicals of the formula

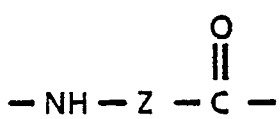

wherein Z is a $C_{2-24}$ alkylene;

with the provision that no more than 5 amino acid residues, or derivatives thereof,designated as "A" above, are bonds, and that the compounds of formula 1 are characterized by a binding constant for ANF receptors of less than about 500 nM; or pharmaceutically acceptable non-toxic salts thereof; comprising the steps of

a) preparing the linear peptide by solid phase sequential or block synthesis, gene cloning or a combination thereof, and

b) subjecting the linear peptide to oxidative coupling.

2. A process for preparing a compound of the formula

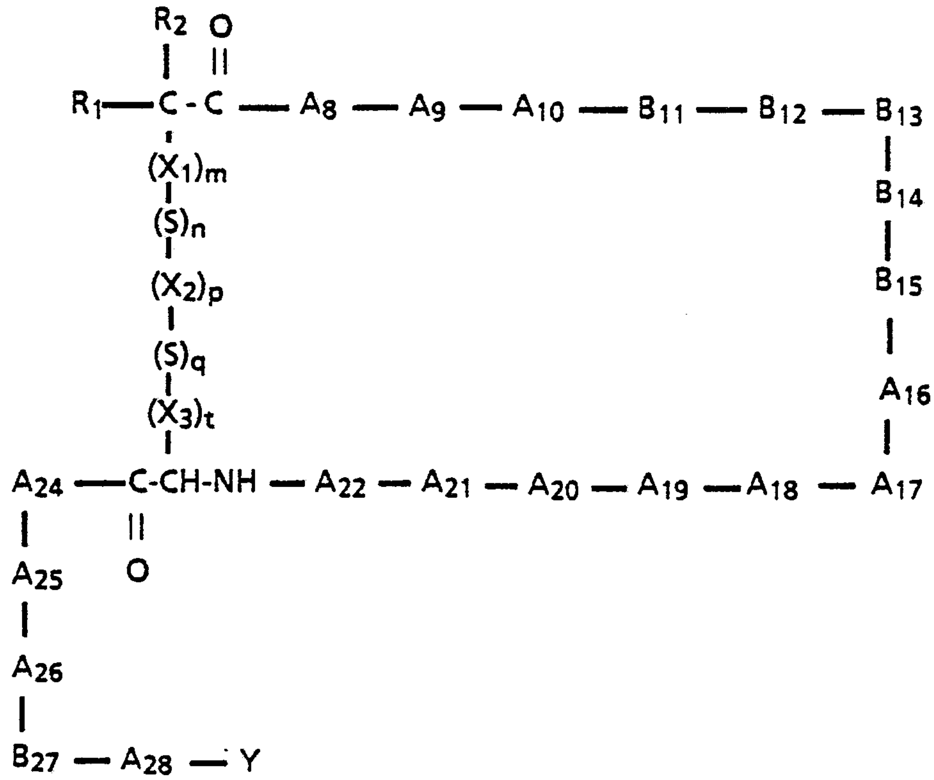

wherein,

$R_1$ is hydrogen, $C_{1-6}$ alkyl, or a radical of the formula

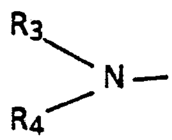

or

$R_3—R_5—NH—$

wherein

$R_3$ and $R_4$ are each independently hydrogen or an amino protecting group;

$R_5$ is a polypeptide of 1 to 10 amino acid residues,

$R_2$ is hydrogen or $C_{1-6}$ alkyl with the provision that when $R_1$ and $R_2$ are each alkyl they can be independent chains or they can be taken together with the carbon atom to which they are attached to form a ($C_3$-$C_{13}$)cyclic alkyl;

$X_1$, $X_2$, and $X_3$ are each independently $C_{1-8}$ alkylene radicals;

m, n, p, q, and t are each independently integers 0 or 1;

$A_8$ is a bond, Phe, N-substituted $C_{1-4}$ alkyl Phe, α-C substituted $C_{1-4}$ alkyl Phe, or D-Phe;

$A_9$ is a bond, Gly or Ala, N-substituted $C_{1-4}$ alkyl -Gly or -Ala, α-C substituted $C_{1-4}$ alkyl -Gly or -Ala, or D-Ala;

$A_{10}$ is a bond, Gly or Ala, N-substituted $C_{1-4}$ alkyl -Gly or -Ala, α-C substituted $C_{1-4}$ alkyl -Gly or -Ala, or D-Ala;

$B_{11}$ is a bond, Arg, N-substituted $C_{1-4}$ alkyl Arg, α-C-substituted $C_{1-4}$ alkyl Arg, or D-Arg;

$B_{12}$ is a bond, Ile, Nle, or Met; N-substituted $C_{1-4}$ alkyl -Ile, -Nle, or -Met; α-C-substituted $C_{1-4}$ alkyl -Ile, -Nle, or -Met; or D-Ile, D-Nle, or D-Met;

$B_{13}$ is a bond, Asp or Glu, N-substituted $C_{1-4}$ alkyl -Asp or -Glu, α-C-substituted $C_{1-4}$ alkyl -Asp or -Glu, or D-Asp or D-Glu;

$B_{14}$ is a bond, Arg, N-substituted $C_{1-4}$ alkyl Arg, α-C-substituted $C_{1-4}$ alkyl Arg, or D-Arg;

$B_{15}$ is a bond, Ile, Nle, or Met; N-substituted $C_{1-4}$ alkyl -Ile, -Nle, or -Met; α-C-substituted $C_{1-4}$ alkyl -Ile, -Nle, or -Met; or D-Ile, D-Nle, or D-Met;

$A_{16}$ is a bond, Gly or Ala, N-substituted $C_{1-4}$ alkyl -Gly or -Ala, α-C substituted $C_{1-4}$ alkyl -Gly or -Ala, or

D-Ala;

$A_{17}$ is a bond, Gly or Ala, N-substituted $C_{1-4}$ alkyl -Gly or -Ala, α-C substituted $C_{1-4}$ alkyl -Gly or -Ala, or D-Ala;

$A_{18}$ is a bond, Gln or Asn, N-substituted $C_{1-4}$ alkyl -Gln or -Asn, α-C substituted $C_{1-4}$ alkyl -Gln or -Asn, or D-Gln or D-Asn;

$A_{19}$ is a bond, Ser or Thr, N-substituted $C_{1-4}$ alkyl -Ser or -Thr, α-C substituted $C_{1-4}$ alkyl -Ser or -Thr, or D-Ser or D-Thr;

$A_{20}$ is a bond, Gly or Ala, N-substituted $C_{1-4}$ alkyl -Gly or -Ala, α-C substituted $C_{1-4}$ alkyl -Gly or -Ala, or D-Ala;

$A_{21}$ is a bond, Leu or Nle, N-substituted $C_{1-4}$ alkyl -Leu or -Nle, α-C substituted $C_{1-4}$ alkyl -Leu or -Nle, or D-Leu or D-Nle;

$A_{22}$ is a bond, Gly or Ala, N-substituted $C_{1-4}$ alkyl -Gly or -Ala, α-C substituted $C_{1-4}$ alkyl -Gly or -Ala, or D-Ala;

$A_{24}$ is a bond, Asn or Gln, N-substituted $C_{1-4}$ alkyl -Asn or -Gln, α-C-substituted $C_{1-4}$ alkyl Asn or Gln, or D-Asn or D-Gln;

$A_{25}$ is a bond, Ser, N-substituted $C_{1-4}$ alkyl Ser, α-C-substituted $C_{1-4}$ alkyl Ser, or D-Ser;

$A_{26}$ is a bond, Phe, N-substituted $C_{1-4}$ alkyl Phe, α-C-substituted $C_{1-4}$ alkyl Phe, or D-Phe;

$A_{27}$ is a bond, Arg, N-substituted $C_{1-4}$ alkyl Arg, α-C-substituted $C_{1-4}$ alkyl Arg, or D-Arg;

$A_{28}$ is a bond, Tyr, N-substituted $C_{1-4}$ alkyl Tyr, α-C-substituted $C_{1-4}$ alkyl Tyr, or D-Tyr;

Y is -OH, $C_{1-6}$ alkoxy, amino, mono- or di- $C_{1-4}$ alkyl substituted amino;

and wherein one or more amino acid residues,or derivatives thereof, designated as "A" above, are replaced by one or more divalent radicals of the formula

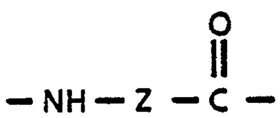

wherein Z is a $C_{2-24}$ alkylene;

with the provision that no more than 5 amino acid residues, or derivatives thereof, designated as "A" above, are bonds; or pharmaceutically acceptable non-toxic salts thereof; comprising the steps of

a) preparing the linear peptide by solid phase sequential or block synthesis, gene cloning or a combination thereof, and

b) subjecting the linear peptide to oxidative coupling.

3. A solid phase sequential or block process for preparing a compound of the formula

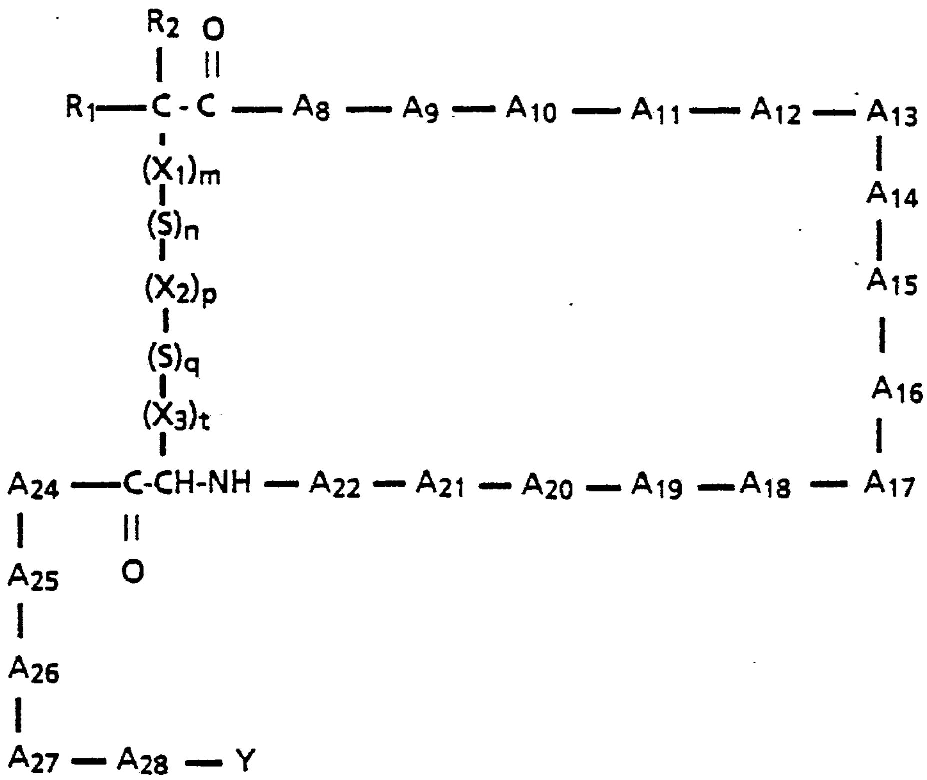

wherein,

$R_1$ is hydrogen, $C_{1-6}$ alkyl, or a radical of the formula

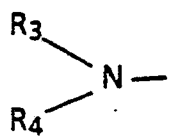

or

$R_3 — R_5 — NH —$

wherein

$R_3$ and $R_4$ are each independently hydrogen or an amino protecting group;

$R_5$ is a polypeptide of 1 to 10 amino acid residues,

$R_2$ is hydrogen or $C_{1-6}$ alkyl with the provision that when $R_1$ and $R_2$ are each alkyl they can be independent chains or they can be taken together with the carbon atom to which they are attached to form a ($C_3$-$C_{13}$)cyclic alkyl;

$X_1$, $X_2$, and $X_3$ are each independently $C_{1-8}$ alkylene radicals;

m, n, p, q, and t are each independently integers 0 or 1;

$A_8$ is a bond, Phe, N-substituted $C_{1-4}$ alkyl Phe, $\alpha$-C substituted $C_{1-4}$ alkyl Phe, or D-Phe;

$A_9$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{10}$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{11}$ is a bond, Arg, N-substituted $C_{1-4}$ alkyl Arg, $\alpha$-C-substituted $C_{1-4}$ alkyl Arg, or D-Arg;

$A_{12}$ is a bond, Ile, Nle, or Met; N-substituted $C_{1-4}$ alkyl -Ile, -Nle, or -Met; $\alpha$-C-substituted $C_{1-4}$ alkyl -Ile, -Nle, or -Met; or D-Ile, D-Nle, or D-Met;

$A_{13}$ is a bond, Asp or Glu, N-substituted $C_{1-4}$ alkyl -Asp or -Glu, $\alpha$-C-substituted $C_{1-4}$ alkyl -Asp or -Glu, or D-Asp or D-Glu;

$A_{14}$ is a bond, Arg, N-substituted $C_{1-4}$ alkyl Arg, $\alpha$-C-substituted $C_{1-4}$ alkyl Arg, or D-Arg;

$A_{15}$ is a bond, Ile, Nle, or Met, N-substituted $C_{1-4}$ alkyl -Ile, -Nle, or -Met, $\alpha$-C-substituted $C_{1-4}$ alkyl -Ile, -Nle, or -Met, or D-Ile, D-Nle, or D-Met;

$A_{16}$ is a bond, Gly or Ala, N-substituted $C_{1-4}$ alkyl -Gly or -Ala, $\alpha$-C substituted $C_{1-4}$ alkyl -Gly or -Ala, or

D-Ala;

$A_{17}$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{18}$ is a bond, Gln or Asn, N-substituted $C_{1-4}$ alkyl -Gln or -Asn, $\alpha$-C substituted $C_{1-4}$ alkyl -Gln or -Asn, or D-Gln or D-Asn;

$A_{19}$ is a bond, Ser or Thr, N-substituted $C_{1-4}$ alkyl -Ser or -Thr, $\alpha$-C substituted $C_{1-4}$ alkyl -Ser or -Thr, or D-Ser or D-Thr;

$A_{20}$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{21}$ is a bond, Leu or Nle, N-substituted $C_{1-4}$ alkyl -Leu or -Nle, $\alpha$-C substituted $C_{1-4}$ alkyl -Leu or -Nle, or D-Leu or D-Nle;

$A_{22}$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{24}$ is a bond, Asn or Gln, N-substituted $C_{1-4}$ alkyl Asn or Gln, $\alpha$-C-substituted $C_{1-4}$ alkyl Asn or Gln, or D-Asn or D-Gln;

$A_{25}$ is a bond, Ser, N-substituted $C_{1-4}$ alkyl Ser, $\alpha$-C-substituted $C_{1-4}$ alkyl Ser, or D-Ser; $A_{26}$ is a bond, Phe, N-substituted $C_{1-4}$ alkyl Phe, $\alpha$-C-substituted $C_{1-4}$ alkyl Phe, or D-Phe;

$A_{27}$ is a bond, Arg, N-substituted $C_{1-4}$ alkyl Arg, $\alpha$-C-substituted $C_{1-4}$ alkyl Arg, or D-Arg;

$A_{28}$ is a bond, Tyr, N-substituted $C_{1-4}$ alkyl Tyr, $\alpha$-C-substituted $C_{1-4}$ alkyl Tyr, or D-Tyr;

Y is -OH, $C_{1-6}$ alkoxy, amino, mono- or di- $C_{1-4}$ alkyl substituted amino;

and wherein one or more amino acid residues,or derivatives thereof, designated as "A" above, are replaced by one or more divalent radicals of the formula

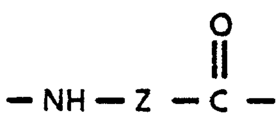

wherein Z is a $C_{2-24}$ alkylene;

with the provision that no more than 5 amino acid residues, or derivatives thereof,designated as "A" above, are bonds, and that the compounds of formula 1 are characterized by a binding constant for ANF receptors of less than about 500 nM; or pharmaceutically acceptable non-toxic salts thereof; comprising the steps of

a) binding a suitably protected amino acid which is desired on the carboxy terminal end of said compound to a resin support,

b) subsequently binding the other $\alpha$-amino protected amino acids to the terminal amino group of the growing peptidic chain which has meanwhile been exposed by removing its $\alpha$-amino protecting group, and

c) subjecting the linear peptide to an oxidative coupling.

4. A solid phase sequential or block process for preparing a compound of the formula

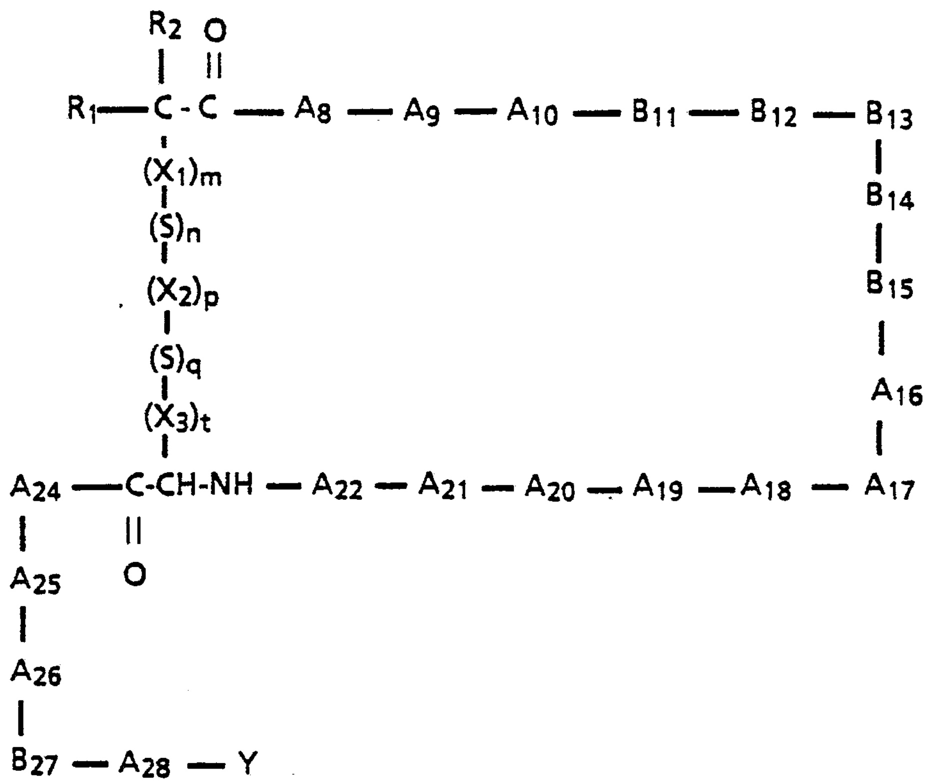

wherein,

$R_1$ is hydrogen, $C_{1-6}$ alkyl, or a radical of the formula

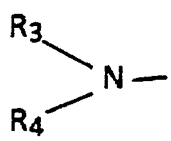

or

$R_3 - R_5 - NH -$

wherein

$R_3$ and $R_4$ are each independently hydrogen or an amino protecting group;

$R_5$ is a polypeptide of 1 to 10 amino acid residues,

$R_2$ is hydrogen or $C_{1-6}$ alkyl with the provision that when $R_1$ and $R_2$ are each alkyl they can be independent chains or they can be taken together with the carbon atom to which they are attached to form a ($C_3$-$C_{13}$)cyclic alkyl;

$X_1$, $X_2$, and $X_3$ are each independently $C_{1-8}$ alkylene radicals;

m, n, p, q, and t are each independently integers 0 or 1;

$A_8$ is a bond, Phe, N-substituted $C_{1-4}$ alkyl Phe, α-C substituted $C_{1-4}$ alkyl Phe, or D-Phe;

$A_9$ is a bond, Gly or Ala, N-substituted $C_{1-4}$ alkyl -Gly or -Ala, α-C substituted $C_{1-4}$ alkyl -Gly or -Ala, or D-Ala;

$A_{10}$ is a bond, Gly or Ala, N-substituted $C_{1-4}$ alkyl -Gly or -Ala, α-C substituted $C_{1-4}$ alkyl -Gly or -Ala, or D-Ala;

$B_{11}$ is a bond, Arg, N-substituted $C_{1-4}$ alkyl Arg, α-C-substituted $C_{1-4}$ alkyl Arg, or D-Arg;

$B_{12}$ is a bond, Ile, Nle, or Met; N-substituted $C_{1-4}$ alkyl -Ile, -Nle, or -Met; α-C-substituted $C_{1-4}$ alkyl -Ile, -Nle, or -Met; or D-Ile, D-Nle, or D-Met;

$B_{13}$ is a bond, Asp or Glu, N-substituted $C_{1-4}$ alkyl -Asp or -Glu, α-C-substituted $C_{1-4}$ alkyl -Asp or -Glu, or D-Asp or D-Glu;

$B_{14}$ is a bond, Arg, N-substituted $C_{1-4}$ alkyl Arg, α-C-substituted $C_{1-4}$ alkyl Arg, or D-Arg;

$B_{15}$ is a bond, Ile, Nle, or Met; N-substituted $C_{1-4}$ alkyl -Ile, -Nle, or -Met; α-C-substituted $C_{1-4}$ alkyl -Ile,

-Nle, or -Met; or D-Ile, D-Nle, or D-Met;

$A_{16}$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{17}$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{18}$ is a bond, Gln or Asn, N-substituted $C_{1-4}$ alkyl -Gln or -Asn, $\alpha$-C substituted $C_{1-4}$ alkyl -Gln or -Asn, or D-Gln or D-Asn;

$A_{19}$ is a bond, Ser or Thr, N-substituted $C_{1-4}$ alkyl -Ser or -Thr, $\alpha$-C substituted $C_{1-4}$ alkyl -Ser or -Thr, or D-Ser or D-Thr;

$A_{20}$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{21}$ is a bond, Leu or Nle, N-substituted $C_{1-4}$ alkyl -Leu or -Nle, $\alpha$-C substituted $C_{1-4}$ alkyl -Leu or -Nle, or D-Leu or D-Nle;

$A_{22}$ is a bond, Gly or Ala, N-substituted $C_{1-4}$ alkyl -Gly or -Ala, $\alpha$-C substituted $C_{1-4}$ alkyl -Gly or -Ala, or D-Ala;

$A_{24}$ is a bond, Asn or Gln, N-substituted $C_{1-4}$ alkyl -Asn or -Gln, $\alpha$-C-substituted $C_{1-4}$ alkyl Asn or Gln, or D-Asn or D-Gln;

$A_{25}$ is a bond, Ser, N-substituted $C_{1-4}$ alkyl Ser, $\alpha$-C-substituted $C_{1-4}$ alkyl Ser, or D-Ser;

$A_{26}$ is a bond, Phe, N-substituted $C_{1-4}$ alkyl Phe, $\alpha$-C-substituted $C_{1-4}$ alkyl Phe, or D-Phe;

$A_{27}$ is a bond, Arg, N-substituted $C_{1-4}$ alkyl Arg, $\alpha$-C-substituted $C_{1-4}$ alkyl Arg, or D-Arg;

$A_{28}$ is a bond, Tyr, N-substituted $C_{1-4}$ alkyl Tyr, $\alpha$-C-substituted $C_{1-4}$ alkyl Tyr, or D-Tyr;

Y is -OH, $C_{1-6}$ alkoxy, amino, mono- or di- $C_{1-4}$ alkyl substituted amino;

and wherein one or more amino acid residues,or derivatives thereof, designated as "A" above, are replaced by one or more divalent radicals of the formula

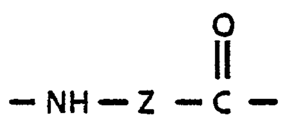

wherein Z is a $C_{2-24}$ alkylene;

with the provision that no more than 5 amino acid residues, or derivatives thereof, designated as "A" above, are bonds; or pharmaceutically acceptable non-toxic salts thereof; comprising the steps of

a) binding a suitably protected amino acid which is desired on the carboxy terminal end of said compound to a resin support,

b) subsequently binding the other $\alpha$-amino protected amino acids to ther terminal amino group of the growing peptidic chain which has meanwhile been exposed by removing its $\alpha$-amino protecting group, and

c) subjecting the linear peptide to an oxidative coupling.

5. A process for preparing a compound according to claim 1, 2, 3 or 4 which is a compound wherein $R_5$ is Ser, Ser-Ser, Arg-Ser-Ser, Arg-Arg-Ser-Ser, Leu-Arg-Arg-Ser-Ser, or Ser-Leu-Arg-Arg-Ser-Ser.

6. A process for preparing a compound according to claim 1, 2, 3 or 4 which is a compound wherein $X_1$ and $X_3$ are methylene; m, n, q, and t are 1; and p is 0.

7. A process for preparing a compound according to claim 1, 2, 3 or 4 which is a compound wherein $X_1$ and $X_3$ are methylene; $X_2$ is ethylene; m, p, and t are 1: and n and q are 0.

8. A process for preparing a compound according to claim 1, 2, 3 or 4 which is a compound wherein $X_1$ is a cyclopentylene; $X_3$ is methylene; m, n, q, and t are 1; and p is 0.

9. A process for preparing a compound according to claim 1, 2, 3 or 4 which is a compound wherein $X_1$ is a cyclohexylene; $X_3$ is methylene; m, n, q, and t are 1; and p is 0.

10. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{17}$ is Ala, $A_{18}$ is Gln, $A_{19}$ is Ser, $A_{20}$ is Gly, $A_{21}$ is Leu, $A_{22}$ is Gly, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2-S-S-CH_2-$, the divalent residue of aminooctanoic acid is a spacer group occupying positions 24 through 26, and Y is $-NH_2$.

11. A process for preparing a compound according to claim 1, 2, 3 or 4 which is a compound wherein $A_{11}$ is Arg, $A_{12}$ is Ile, $A_{13}$ is Asp, $A_{14}$ is Arg, $A_{15}$ is Ile, $A_{16}$ is Gly, $A_{17}$ is Ala, $A_{18}$ is Gln, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 8 through 10, and Y is $-NH_2$.

12. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 17 through 19, and Y is $-NH_2$.

13. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{17}$ is Ala, $A_{18}$ is Gln, $A_{19}$ is Ser, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 20 through 22, and Y is $-NH_2$.

14. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{18}$ is Gln, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 15 through 17, and Y is $-NH_2$.

15. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 16 through 18, and Y is $-NH_2$.

16. A process for preparing a compound according to claim 1, 2, 3 or 4 which is a compound wherein $A_8$ is Phe, $A_9$ is Gly, $A_{10}$ is Gly, $A_{14}$ is Arg, $A_{15}$ is Ile, $A_{16}$ is Gly, $A_{17}$ is Ala, $A_{18}$ is Gln, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 11 through 13, and Y is $-NH_2$.

17. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is Ile, $A_{13}$ is Asp, $A_{17}$ is Ala, $A_{18}$ is Gln, $A_{19}$ is Ser, $A_{20}$ is Gly, $A_{21}$ is Leu, $A_{22}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 14 through 16, and Y is $-NH_2$.

18. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{24}$ is Asn, $A_{25}$ is Ser, $A_{26}$ is Phe, $A_{27}$ is Arg, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, two divalent residues of aminooctanoic acid are spacer groups occupying positions 17 through 19 and 20 through 22, and Y is $-NH_2$.

19. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is Ile, $A_{13}$ is Asp, $A_{14}$ is Arg, $A_{15}$ is Ile, $A_{16}$ is Gly, $A_{17}$ is Ala, $A_{18}$ is Gln, $A_{19}$ is Ser, $A_{20}$ is Gly, $A_{21}$ is Leu, $A_{22}$ is Gly, $A_{24}$ is Asn, $A_{28}$ is Tyr, $R_1$ is H-Ser-Ser-NH-, $R_2$ is H, $(X_1)_m$-$(S)_n$-$(X_2)_p$-$(S)_q$-$(X_3)_t$- is $-CH_2$-S-S-$CH_2$-, the divalent residue of aminooctanoic acid is a spacer group occupying positions 25 through 27, and Y is $-NH_2$.

20. A process for preparing a compound according to claim 1, 2, 3 or 4 which is a compound wherein $A_8$ is Phe, $A_{11}$ is Arg, $A_{12}$ is N-Me-Ile, $A_{13}$ is Asp, $A_{14}$ is Arg, $A_{15}$ is N-Me-Ile, $A_{27}$ is D-Arg, $R_1$ is H, $R_2$ is H, $(X_1)_m$-$(S)_n$-$(X_2)_p$-$(S)_q$-$(X_3)_t$- is $-CH_2$-S-S-$CH_2$-, the divalent residue of aminopentanoic acid is a spacer group occupying positions 9 and 10, the divalent residue of aminoundecanoic acid is a spacer group occupying positions 16 through 19, two divalent residues of aminooctanoic acid are spacer groups occupying positions 20 through 22 and 24 through 26, and Y is $-NH_2$.

21. A process for preparing a compound according to claim 1, 2, 3 or 4 which is a compound wherein $A_8$ is Phe, $A_9$ is Gly, $A_{10}$ is Gly, $A_{11}$ is Arg, $A_{12}$ is N-Me-Ile, $A_{13}$ is Asp, $A_{14}$ is Arg, $A_{15}$ is N-Me-Ile, $A_{27}$ is D-Arg, $A_{28}$ is Tyr, $R_1$ is H, $R_2$ is H, $(X_1)_m$-$(S)_n$-$(X_2)_p$-$(S)_q$-$(X_3)_t$- is $-CH_2$-S-S-$CH_2$-, the divalent residue of aminoundecanoic acid is a spacer group occupying positions 16 through 19, two divalent residues of aminooctanoic acid are spacer groups occupying positions 20 through 22 and 24 through 26, and Y is $-NH_2$.

22. A process for preparing a compound according to claim 1, 2, 3 or 4 which is a compound wherein $A_8$ is N-Me-Phe, $A_9$ is Gly, $A_{10}$ is Gly, $A_{11}$ is Arg, $A_{12}$ is N-Me-Ile, $A_{13}$ is Asp, $A_{14}$ is Arg, $A_{15}$ is N-Me-Ile, $A_{27}$ is D-Arg, $A_{28}$ is Tyr, $R_1$ is H-Ser-Ser-NH, $R_2$ is H, $(X_1)_m-(S)_n-(X_2)_p-(S)_q-(X_3)_t-$ is $-CH_2-S-S-CH_2-$, the divalent residues of aminoundecanoic acid is a spacer group occupying positions 16 through 19, two divalent residues of aminooctanoic acid are spacer groups occupying positions 20 through 22 and 24 through 26, and Y is $-NH_2$.

23. A process for preparing a compound according to claim 1, 2, 3 or 4 which is a compound wherein $A_{11}$ is Arg, $A_{12}$ is Ile, $A_{13}$ is Asp, $A_{14}$ is Arg, $A_{15}$ is Ile, $A_{16}$ is Gly, $A_{27}$ is Arg, $A_{28}$ is Tyr, $R_1$ is H-Ser-Ser-NH, $R_2$ is H, $(X_1)_m-(S)_n-(X_2)_p-(S)_q-(X_3)_t-$ is $-CH_2-S-S-CH_2-$, four divalent residues of aminooctanoic acid are spacer groups occupying positions 8 through 10, 17 through 19, 20 through 22, and 24 through 26, and Y is $-NH_2$.

24. Use of a compound of any one of the preceding claims for preparing a medicament for treating abnormalities in fluid, electrolyte, blood pressure, intraocular pressure, renin, or aldosterone homeostasis.

25. Use according to claim 24 wherein the medicament is used for treating hypertension, renal diseases, hyperaldosteronemia, cardiac hypertrophy, glaucoma, and congestive heart failure.